# NEUE EUROPÄISCHE PATENTSCHRIFT

(11) **EP 1 723 945 B2**
(45) Veröffentlichungstag und Bekanntmachung des Hinweises auf die Entscheidung über den Einspruch: **13.12.2017**
(45) Hinweis auf die Patenterteilung: 20.03.2013
(21) Anmeldenummer: 05024878.0
(22) Anmeldetag: 15.11.2005
(51) Int. Cl.: A61K 8/36, A61K 8/368, A61K 8/81, A61Q 5/02, A61K 8/49

(54) **Haarreinigungsmittel mit optimiertem Fliessverhalten**
Hair cleaning compositions with optimised rheological behaviour
Compositions de nettoyage des cheveux à écoulement amélioré

(30) Priorität: 21.03.2005 DE 102005013489
(43) Veröffentlichungstag der Anmeldung: 22.11.2006
(73) Patentinhaber: Henkel AG & Co. KGaA, 40589 Düsseldorf (DE)
(72) Erfinder: Goddinger, Dieter, 25336 Klein Nordende (DE); Schröder, Thomas, 22395 Hamburg (DE)

(56) Entgegenhaltungen:
- EP-A- 1 457 194
- WO-A-01/76552
- DE-A1- 4 129 986
- DE-A1- 19 808 395
- DE-A1- 19 916 335
- RAMIREZ A ET AL: "RHEOLOGICAL STUDY OF CARBOPOL ULTREZ TM 10 HYDROALCOHOLIC GELS, I: FLOW AND THIXOTROPIC BEHAVIOR AS A FUNCTION OF PH AND POLYMER CONCENTRATION" PHARMAZIE, DIE, GOVI VERLAG, ESCHBORN, DE, Bd. 54, Nr. 6, Juni 1999 (1999-06), Seiten 444-447, XP000828548 ISSN: 0031-7144

## Beschreibung

Die Erfindung betrifft Haarreinigungsmittel, welche Tensid(e), eine Säurekonservierung und bestimmte Viskositätsregulantien enthalten sowie die Verwendung dieser Mittel zur Reinigung und/oder Pflege von Haut und Haar.

Die kosmetische Behandlung von Haut und Haaren ist ein wichtiger Bestandteil der menschlichen Körperpflege. So wird menschliches Haar heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle.

Verbraucher erwarten von einem kosmetischen Produkt unter allen Anwendungsbedingungen eine konstante Produktleistung. Bei tensidischen Reinigungsformulierungen wie beispielsweise Shampoos, Duschgelen, Badeschäumen usw. ist die Viskosität jedoch stark temperaturabhängig, wobei die Viskosität im Regelfall mit steigender Temperatur sinkt. In der Folge wird ein entsprechendes Produkt vom Verbraucher im Sommer als "zu dünn" im Winter als zu zähflüssig empfunden. Darüber hinaus erwartet der Verbraucher unabhängig von seinem Standort und den dort herrschenden klimatischen Bedingungen identische Produkteigenschaften.

Die in Temperaturbereichen, die normalen Anwendungstemperaturen der o.g. Mittel entsprechen, stabile Viskositätseinstellung gelingt mit bestimmten Polymeren. Der Einsatz dieser Polymere schränkt aber die Formulierungsfreiheit ein, da die meisten Viskositätsregler empfindlich auf die Elektrolytkonzentration der Mittel und auf deren pH-Wert reagieren. Stabil verdickte Formulierungen lassen sich daher nur im neutralen pH-Wert-Bereich herstellen.

Diese Mittel haben wiederum den Nachteil, daß sie zur Konservierung gegen den Befall mit Mikroorganismen nur mit solchen Konservierungsmitteln geschützt werden können, die von manchen Verbrauchern kritisch betrachtet werden und unerwünscht sind, beispielsweise weil sie Formaldehyd abspalten.

Die internationale Patentanmeldung WO 01/76552 A1 offenbart stabile wäßrige Tensid-Zusammensetzungen, die 1 bis 80 Gew.-% mindestens eines tensids, 0,1 bis 10 Gew.-% mindestens eins Polymers zur Voskositätsregulierung und 0,1 bis 20 Gew.-% mindestens einer organischen Säure zur pH-Regulierung enthalten. Die in dieser Schrift offenbarten Beispielzusammensetzungen enthalten Phenoxyethanol oder parabene als Konservierungsmittel.

Es bestand daher die Aufgabe, Reinigungsmittelzusammensetzungen bereitzustellen, die auch bei einer vom Verbraucher als unkritisch empfundenen Konservierung in weiten Temperaturbereichen viskositätsstabil sind.

Es wurde nun gefunden, daß die Kombination von Säurekonservierungsmitteln mit bestimmten Polymeren die genannten Nachteile überwindet.

Gegenstand der vorliegenden Anmeldung sind in einer ersten Ausführungsform Haarreinigungsmittel, enthaltend bezogen auf ihr Gewicht
a) 5 bis 50 Gew.-% Tensid(e);
b) 0,01 bis 5 Gew.-% einer oder mehrerer als Konservierungsmittel wirksamer organischer Säure(n) aus der Gruppe Salicylsäure, Benzoesäure, Propionsäure, Dehydracetsäure (3-Acetyl-6-methyl-2H-pyran-2,4(3H)-dion) und Sorbinsäure sowie den physiologisch verträglichen wasserlöslichen Salzen dieser Säuren;
c) 0,1 bis 5 Gew.-% mindestens eines Homo- oder Copolymers aus Acrylsäure und/oder Methacrylsäure und/oder Estern dieser Säuren, welches mit Allylethern von Sucrose und/oder Allylethern von Pentaerythrit vernetzt ist,
wobei das Mittel - abgesehen von der Komponente b) - frei von Konservierungsmitteln ist,
wobei die als Konservierungsmittel wirksame Säure ausgewählt ist aus Salicylsäure und ihren physiologisch verträglichen wasserlöslichen Salzen, und wobei es einen pH-Wert von 4,5 bis 5,5 aufweist.

Die erfindungsgemäßen Mittel sind erfindungsgemäß innerhalb eines engen pH-Wert-Bereiches formuliert. Bevorzugte Haarreinigungsmittel sind dabei dadurch gekennzeichnet, daß sie einen pH-Wert von 4,8 bis 5,2, weiter bevorzugt von 4,9 bis 5,1 und insbesondere von 5,0 aufweisen. Sofern im Rahmen der vorliegenden Anmeldung physikalische Meßgrößen genannt werden, bezieht sich die Messung überall dort, wo nichts anderes ausgeführt wird, auf eine Meßtemperatur von 20°C.

Die erfindungsgemäßen Mittel enthalten als Komponente b) eine oder mehrere als Konservierungsmittel wirksame organische Säure(n) aus der Gruppe Salicylsäure, Benzoesäure, Propionsäure, Dehydracetsäure (3-Acetyl-6-methyl-2H-pyran-2,4(3H)-dion) und Sorbinsäure sowie den physiologisch verträglichen wasserlöslichen Salzen dieser Säuren, wobei die als Konservierungsmittel wirksame Säure ausgewählt ist aus Salicylsäure und ihren physiologisch verträglichen wasserlöslichen Salzen.

Weiter bevorzugt sind Haarreinigungsmittel, die mindestens eine als Konservierungsmittel wirksame organische Säure, ausgewählt aus Salicylsäure, sowie den physiologisch verträglichen wasserlöslichen Metallsalzen dieser Säuren, enthalten und mit 0,1 - 10 Gew.-% eines korrespondierenden Säure-Base-Paares so abgepuffert sind, daß sie sowohl direkt nach der Herstellung als auch nach zweimonatiger Lagerung den vorstehend genannten pH-Wert-Kriterien genügen.

Unter physiologisch verträglichen Metallsalzen sind erfindungsgemäß die Natrium-, Kalium-, Magnesium- und gegebenenfalls Calciumsalze der Salicylsäure zu verstehen, sofern diese ausreichend wasserlöslich sind. "Wasserlöslich" bedeutet erfindungsgemäß eine Löslichkeit von mindestens 1 g Salz pro 100 g wässriger Lösung bei 20°C.
Erfindungsgemäß besonders bevorzugt sind die Natrium- und Kalium-Salze.
Die Salicylsäure, Benzoesäure, Propionsäure, Dehydracetsäure und Sorbinsäure und deren genannten Salze werden erfindungsgemäß in Mengen von 0,01 - 5 Gew.-% eingesetzt, wobei bevorzugte erfindungsgemäße Haarreinigungsmittel dadurch gekennzeichnet sind, daß sie die Komponente(n) b) in Mengen von 0,05 bis 4 Gew.-%, vorzugsweise von 0,3 bis 3 Gew.-%, besonders bevorzugt von 0,4 bis 2 Gew.-% und insbesondere von 0,5 bis 1 Gew.-%, jeweils bezogen auf das gesamte Mittel, enthält.

In der Erfindung ist die als Konservierungsmittel wirksame organische Säure ausgewählt aus Salicylsäure und ihren physiologisch verträglichen wasserlöslichen Salzen, insbesondere Natriumsalicylat. Salicylsäure weist neben der konservierenden Wirkung auch kosmetische Wirkeigenschaften auf.

Erfindungsgemäß geeignete korrespondierende Säure-Base-Paare sind wässrige Pufferlösungen, die die erfindungsgemäßen wässrigen Haarreinigungsmittel im gewünschten pH-Bereich stabilisieren. Bevorzugt sind wässrige Lösungen von Citronensäure und ihren Natriumsalzen, insbesondere Trinatriumcitrat, Weinsäure und ihren Kaliumsalzen, insbesondere Kaliumhydrogentartrat, und Phthalsäure und Ihren Kaliumsalzen, insbesondere Kaliumhydrogenphthalat Ein besonders bevorzugtes Puffersystem ist aus Citronensäure und den Natriumcitraten gebildet. Andere Puffersysteme, z.B. Essigsäure/Natriumacetat, sind prinzipiell ebenfalls erfindungsgemäß geeignet. Aufgrund des Essiggeruchs ist ein solcher Puffer allerdings nicht für die Herstellung eines kosmetischen Marktproduktes akzeptabel.

Erfindungsgemäße Haarreinigungsmittel sind dadurch gekennzeichnet, daß sie - abgesehen von der Komponente b) - frei von Konservierungsmitteln sind.

Die erfindungsgemäßen Mittel enthalten als Verdicker ein Homo- oder Copolymer aus Acrylsäure und/oder Methacrylsäure und/oder Estern dieser Säuren, welches mit Allylethern von Sucrose und/oder Allylethern von Pentaerythrit vernetzt ist.

In diese Gruppe fallen beispielsweise
- Polyacrylsäuren, welche mit Allylethern von Sucrose vernetzt sind,
- Polyacrylsäuren, welche mit Allylethern von Pentaerythrit vernetzt sind,
- Polymethacrylsäuren, welche mit Allylethern von Sucrose vernetzt sind,
- Polymethacrylsäuren, welche mit Allylethern von Pentaerythrit vernetzt sind,
- Polyacrylsäureester, welche mit Allylethern von Sucrose vernetzt sind,
- Polyacrylsäureester, welche mit Allylethern von Pentaerythrit vernetzt sind,
- Polymethacrylsäureester, welche mit Allylethern von Sucrose vernetzt sind,
- Polymethacrylsäureester, welche mit Allylethern von Pentaerythrit vernetzt sind
- Copolymere von Acrylsäure und Methacrylsäure, welche mit Allylethern von Sucrose vernetzt sind,
- Copolymere von Acrylsäure und Methacrylsäure, welche mit Allylethern von Pentaerythrit vernetzt sind,
- Copolymere von Acrylsäureestern und Methacrylsäure, welche mit Allylethern von Sucrose vernetzt sind,
- Copolymere von Acrylsäureestern und Methacrylsäure, welche mit Allytethern von Pentaerythrit vernetzt sind,
- Copolymere von Acrylsäure und Methacrylsäureestern, welche mit Allylethern von Sucrose vernetzt sind,
- Copolymere von Acrylsäure und Methacrylsäureestern, welche mit Allylethern von Pentaerythrit vernetzt sind,
- Copolymere von Acrylsäureestern und Methacrylsäureestern, welche mit Allylethern von Sucrose vernetzt sind,
- Copolymere von Acrylsäureestern und Methacrylsäureestern, welche mit Allylethern von Pentaerythrit vernetzt sind,

Bevorzugte erfindungsgemäße Haarreinigungsmittel sind dadurch gekennzeichnet, daß sie als Komponente c) ein Homopolymer von Acrylsäure enthalten, das mit Allylethern von Sucrose und/oder Allylethern von Pentaerythrit vernetzt ist (INCI-Bezeichnung: Carbomer). Solche Polymere sind unter den Bezeichnungen Acrisint® 400, Acrisint® 410, Acrisint® 430, Acritamer® 934, Acritamer® 940, Acritamer® 941, Carbopol® 910, Carbopol® 934, Carbopol® 940, Carbopol® 941, Carbopol® 951, Carbopol® 954, Carbopol® 981, Carbopol® 984, Carbopol® 934P, Synthalen® K, Synthalen® L, Synthalen® M oder Thixol® 100-C im Handel erhältlich.

Bevorzugt sind ebenfalls erfindungsgemäße Haarreinigungsmittel, die als Komponente c) ein Copolymer aus
i. Acrylsäure und/oder Methacrylsäure und/oder Estern dieser Säuren und
ii. Alkylacrylat(en)
   enthalten, das mit Allylethern von Sucrose und/oder Allylethern von Pentaerythrit vemetzt ist (INCI-Bezeichnung: Acrylates/Alkyl Acrylate Crospolymer).
   Unter diesen bevorzugten erfindungsgemäßen Haarreinigungsmitteln sind solche bevorzugt, bei denen das Alkylacrylat 10, 11, 12, 13, 14, 15, 16, 17, 18, 19, 20, 21, 22, 23, 24, 25, 26, 27, 28, 29 oder 30 Kohlenstoffatome in der Kohlenstoffkette des Alkylrestes aufweist. Solche erfindungsgemäß besonders bevorzugten Haarreinigungsmittel sind dadurch gekennzeichnet, daß sie als Komponente c) ein Copolymer aus
iii. Acrylsäure und/oder Methacrylsäure und/oder Estern dieser Säuren und
iv. C₁₀₋₃₀-Alkylacrylat(en)
enthalten, das mit Allylethern von Sucrose und/oder Altylethern von Pentaerythrit vernetzt ist (INCI-Bezeichnung: Acrylates/C10-30 Alkyl Acrylate Crospolymer).

In diese Gruppe fallen beispielsweise
- Copolymere von Acrylsäure und Decylacrylat, welche mit Allylethern von Sucrose vernetzt sind,
- Copolymere von Acrylsäure und Decylacrylat, welche mit Allylethern von Pentaerythrit vernetzt sind,
- Copolymere von Acrylsäure und Undecylacrylat, welche mit Allylethern von Sucrose vernetzt sind,
- Copolymere von Acrylsäure und Undecylacrylat, welche mit Allylethern von Pentaerythrit vernetzt sind,
- Copolymere von Acrylsäure und Dodecylacrylat, welche mit Allylethern von Sucrose vernetzt sind,
- Copolymere von Acrylsäure und Dodecylacrylat, welche mit Allylethern von Pentaerythrit vernetzt sind,
- Copolymere von Acrylsäure und Tridecylacrylat, welche mit Allylethern von Sucrose vernetzt sind,
- Copolymere von Acrylsäure und Tridecylacrylat, welche mit Allylethern von Pentaerythrit vernetzt sind,
- Copolymere von Acrylsäure und Tetradecylacrylat, welche mit Allylethern von Sucrose vernetzt sind,
- Copolymere von Acrylsäure und Tetradecylacrylat, welche mit Allylethern von Pentaerythrit vernetzt sind,
- Copolymere von Acrylsäure und Pentadecylacrylat, welche mit Allylethern von Sucrose vernetzt sind,
- Copolymere von Acrylsäure und Pentadecylacrylat, welche mit Allylethern von Pentaerythrit vernetzt sind,
- Copolymere von Acrylsäure und Hexadecylacrylat, welche mit Allylethern von Sucrose vernetzt sind,
- Copolymere von Acrylsäure und Hexadecylacrylat, welche mit Allylethern von Pentaerythrit vernetzt sind,
- Copolymere von Acrylsäure und Heptadecylacrylat, welche mit Allylethern von Sucrose vernetzt sind,
- Copolymere von Acrylsäure und Heptadecylacrylat, welche mit Allylethern von Pentaerythrit vernetzt sind,
- Copolymere von Acrylsäure und Octadecylacrylat, welche mit Allylethern von Sucrose vernetzt sind,
- Copolymere von Acrylsäure und Octadecylacrylat, welche mit Altylethern von Pentaerythrit vernetzt sind,
- Copolymere von Acrylsäure und Nonadecylacrylat, welche mit Allylethern von Sucrose vernetzt sind,
- Copolymere von Acrylsäure und Nonadecylacrylat, welche mit Allylethern von Pentaerythrit vernetzt sind,
- Copolymere von Acrylsäure und Eicosylacrylat, welche mit Allylethern von Sucrose vernetzt sind,
- Copolymere von Acrylsäure und Eicosylacrylat, welche mit Allylethern von Pentaerythrit vernetzt sind,
- Copolymere von Acrylsäure und Heneicosylacrylat, welche mit Allylethern von Sucrose vernetzt sind,
- Copolymere von Acrylsäure und Heneicosylacrylat, welche mit Allylethern von Pentaerythrit vernetzt sind,
- Copolymere von Acrylsäure und Docosylacrylat, welche mit Allylethern von Sucrose vernetzt sind,
- Copolymere von Acrylsäure und Docosylacrylat, welche mit Allylethern von Pentaerythrit vernetzt sind,
- Copolymere von Acrylsäure und Tricosylacrylat, welche mit Allylethern von Sucrose vernetzt sind,
- Copolymere von Acrylsäure und Tricosylacrylat, welche mit Allylethern von Pentaerythrit vernetzt sind,
- Copolymere von Acrylsäure und Tetracosylacrylat, welche mit Allylethern von Sucrose vernetzt sind,
- Copolymere von Acrylsäure und Tetracosylacrylat, welche mit Allylethern von Pentaerythrit vernetzt sind,
- Copolymere von Acrylsäure und Pentacosylacrylat, welche mit Allylethern von Sucrose vernetzt sind,
- Copolymere von Acrylsäure und Pentacosylacrylat, welche mit Allylethern von Pentaerythrit vernetzt sind,
- Copolymere von Acrylsäure und Hexacosylacrylat, welche mit Allylethern von Sucrose vernetzt sind,
- Copolymere von Acrylsäure und Hexacosylacrylat, welche mit Allylethern von Pentaerythrit vernetzt sind,
- Copolymere von Acrylsäure und Heptacosylacrylat, welche mit Allylethern von Sucrose vernetzt sind,
- Copolymere von Acrylsäure und Heptacosylacrylat, welche mit Allylethern von Pentaerythrit vernetzt sind,
- Copolymere von Acrylsäure und Octacosylacrylat, welche mit Allylethern von Sucrose vernetzt sind,
- Copolymere von Acrylsäure und Octacosylacrylat, welche mit Allylethern von Pentaerythrit vernetzt sind,
- Copolymere von Acrylsäure und Nonacosylacrylat, welche mit Allylethern von Sucrose vernetzt sind,
- Copolymere von Acrylsäure und Nonacosylacrylat, welche mit Allylethern von Pentaerythrit vernetzt sind,
- Copolymere von Acrylsäure und Triacontylacrylat, welche mit Allylethern von Sucrose vernetzt sind,
- Copolymere von Acrylsäure und Triacontylacrylat, welche mit Allylethern von Pentaerythrit vernetzt sind,
- Copolymere von Methacrylsäure und Decylacrylat, welche mit Allylethern von Sucrose vernetzt sind,
- Copolymere von Methacrylsäure und Decylacrylat, welche mit Allylethern von Pentaerythrit vernetzt sind,
- Copolymere von Methacrylsäure und Undecylacrylat, welche mit Allylethern von Sucrose vernetzt sind,
- Copolymere von Methacrylsäure und Undecylacrylat, welche mit Allylethern von Pentaerythrit vernetzt sind,
- Copolymere von Methacrylsäure und Dodecylacrylat, welche mit Allylethern von Sucrose vernetzt sind,
- Copolymere von Methacrylsäure und Dodecylacrylat, welche mit Allylethern von Pentaerythrit vernetzt sind,
- Copolymere von Methacrylsäure und Tridecylacrylat, welche mit Allylethern von Sucrose vernetzt sind,
- Copolymere von Methacrylsäure und Tridecylacrylat, welche mit Allylethern von Pentaerythrit vernetzt sind,
- Copolymere von Methacrylsäure und Tetradecylacrylat, welche mit Allylethern von Sucrose vernetzt sind,
- Copolymere von Methacrylsäure und Tetradecylacrylat, welche mit Allylethern von Pentaerythrit vernetzt sind,
- Copolymere von Methacrylsäure und Pentadecylacrylat, welche mit Allylethern von Sucrose vernetzt sind,
- Copolymere von Methacrylsäure und Pentadecylacrylat, welche mit Allylethern von Pentaerythrit vernetzt sind,
- Copolymere von Methacrylsäure und Hexadecylacrylat, welche mit Allylethern von Sucrose vernetzt sind,
- Copolymere von Methacrylsäure und Hexadecylacrylat, welche mit Altylethern von Pentaerythrit vernetzt sind,
- Copolymere von Methacrylsäure und Heptadecylacrylat, welche mit Allylethern von Sucrose vernetzt sind,
- Copolymere von Methacrylsäure und Heptadecylacrylat, welche mit Allylethern von Pentaerythrit vernetzt sind,
- Copolymere von Methacrylsäure und Octadecylacrylat, welche mit Allylethern von Sucrose vernetzt sind,
- Copolymere von Methacrylsäure und Octadecylacrylat, welche mit Allylethern von Pentaerythrit vernetzt sind,
- Copolymere von Methacrylsäure und Nonadecylacrylat, welche mit Allylethern von Sucrose vernetzt sind,
- Copolymere von Methacrylsäure und Nonadecylacrylat, welche mit Allylethern von Pentaerythrit vernetzt sind,
- Copolymere von Methacrylsäure und Eicosylacrylat, welche mit Allylethern von Sucrose vernetzt sind,
- Copolymere von Methacrylsäure und Eicosylacrylat, welche mit Allylethern von Pentaerythrit vernetzt sind,
- Copolymere von Methacrylsäure und Heneicosylacrylat, welche mit Allylethern von Sucrose vernetzt sind,
- Copolymere von Methacrylsäure und Heneicosylacrylat, welche mit Allylethern von Pentaerythrit vernetzt sind,
- Copolymere von Methacrylsäure und Docosylacrylat, welche mit Allylethern von Sucrose vernetzt sind,
- Copolymere von Methacrylsäure und Docosylacrylat, welche mit Allylethern von Pentaerythrit vernetzt sind,
- Copolymere von Methacrylsäure und Tricosylacrylat, welche mit Allylethern von Sucrose vernetzt sind,
- Copolymere von Methacrylsäure und Tricosylacrylat, welche mit Allylethern von Pentaerythrit vernetzt sind,
- Copolymere von Methacrylsäure und Tetracosylacrylat, welche mit Allylethern von Sucrose vernetzt sind,
- Copolymere von Methacrylsäure und Tetracosylacrylat, welche mit Allylethern von Pentaerythrit vernetzt sind,
- Copolymere von Methacrylsäure und Pentacosylacrylat, welche mit Allylethern von Sucrose vernetzt sind,
- Copolymere von Methacrylsäure und Pentacosylacrylat, welche mit Allylethern von Pentaerythrit vernetzt sind,
- Copolymere von Methacrylsäure und Hexacosylacrylat, welche mit Allylethern von Sucrose vernetzt sind,
- Copolymere von Methacrylsäure und Hexacosylacrylat, welche mit Allylethern von Pentaerythrit vernetzt sind,
- Copolymere von Methacrylsäure und Heptacosylacrylat, welche mit Altylethern von Sucrose vernetzt sind,
- Copolymere von Methacrylsäure und Heptacosylacrylat, welche mit Allylethern von Pentaerythrit vernetzt sind,
- Copolymere von Methacrylsäure und Octacosylacrylat, welche mit Allylethern von Sucrose vernetzt sind,
- Copolymere von Methacrylsäure und Octacosylacrylat, welche mit Allylethern von Pentaerythrit vernetzt sind,
- Copolymere von Methacrylsäure und Nonacosylacrylat, welche mit Allylethern von Sucrose vernetzt sind,
- Copolymere von Methacrylsäure und Nonacosylacrylat, welche mit Allylethern von Pentaerythrit vernetzt sind,
- Copolymere von Methacrylsäure und Triacontylacrylat, welche mit Allylethern von Sucrose vernetzt sind,
- Copolymere von Methacrylsäure und Triacontylacrylat, welche mit Allylethern von Pentaerythrit vernetzt sind,
- Copolymere von Acrylsäure und Methacrylsäure und Decylacrylat, welche mit Allylethern von Sucrose vernetzt sind,
- Copolymere von Acrylsäure und Methacrylsäure und Decylacrylat, welche mit Allylethern von Pentaerythrit vernetzt sind,
- Copolymere von Acrylsäure und Methacrylsäure und Undecylacrylat, welche mit Allylethern von Sucrose vernetzt sind,
- Copolymere von Acrylsäure und Methacrylsäure und Undecylacrylat, welche mit Allylethern von Pentaerythrit vernetzt sind,
- Copolymere von Acrylsäure und Methacrylsäure und Dodecylacrylat, welche mit Allylethern von Sucrose vernetzt sind,
- Copolymere von Acrylsäure und Methacrylsäure und Dodecylacrylat, welche mit Allylethern von Pentaerythrit vernetzt sind,
- Copolymere von Acrylsäure und Methacrylsäure und Tridecylacrylat, welche mit Allylethern von Sucrose vernetzt sind,
- Copolymere von Acrylsäure und Methacrylsäure und Tridecylacrylat, welche mit Allylethern von Pentaerythrit vernetzt sind,
- Copolymere von Acrylsäure und Methacrylsäure und Tetradecylacrylat, welche mit Allylethern von Sucrose vernetzt sind,
- Copolymere von Acrylsäure und Methacrylsäure und Tetradecylacrylat, welche mit Allylethern von Pentaerythrit vernetzt sind,
- Copolymere von Acrylsäure und Methacrylsäure und Pentadecylacrylat, welche mit Allylethern von Sucrose vernetzt sind,
- Copolymere von Acrylsäure und Methacrylsäure und Pentadecylacrylat, welche mit Allylethern von Pentaerythrit vernetzt sind,
- Copolymere von Acrylsäure und Methacrylsäure und Hexadecylacrylat, welche mit Allylethern von Sucrose vernetzt sind,
- Copolymere von Acrylsäure und Methacrylsäure und Hexadecylacrylat, welche mit Allylethern von Pentaerythrit vernetzt sind,
- Copolymere von Acrylsäure und Methacrylsäure und Heptadecylacrylat, welche mit Allylethern von Sucrose vernetzt sind,
- Copolymere von Acrylsäure und Methacrylsäure und Heptadecylacrylat, welche mit Allylethern von Pentaerythrit vernetzt sind,
- Copolymere von Acrylsäure und Methacrylsäure und Octadecylacrylat, welche mit Allylethern von Sucrose vernetzt sind,
- Copolymere von Acrylsäure und Methacrylsäure und Octadecylacrylat, welche mit Allylethern von Pentaerythrit vernetzt sind,
- Copolymere von Acrylsäure und Methacrylsäure und Nonadecylacrylat, welche mit Allylethern von Sucrose vernetzt sind,
- Copolymere von Acrylsäure und Methacrylsäure und Nonadecylacrylat, welche mit Allylethern von Pentaerythrit vernetzt sind,
- Copolymere von Acrylsäure und Methacrylsäure und Eicosylacrylat, welche mit Allylethern von Sucrose vernetzt sind,
- Copolymere von Acrylsäure und Methacrylsäure und Eicosylacrylat, welche mit Allylethern von Pentaerythrit vernetzt sind,
- Copolymere von Acrylsäure und Methacrylsäure und Heneicosylacrylat, welche mit Allylethern von Sucrose vernetzt sind,
- Copolymere von Acrylsäure und Methacrylsäure und Heneicosylacrylat, welche mit Allylethern von Pentaerythrit vernetzt sind,
- Copolymere von Acrylsäure und Methacrylsäure und Docosylacrylat, welche mit Allylethern von Sucrose vernetzt sind,
- Copolymere von Acrylsäure und Methacrylsäure und Docosylacrylat, welche mit Allylethern von Pentaerythrit vernetzt sind,
- Copolymere von Acrylsäure und Methacrylsäure und Tricosylacrylat, welche mit Allylethern von Sucrose vernetzt sind,
- Copolymere von Acrylsäure und Methacrylsäure und Tricosylacrylat, welche mit Allylethern von Pentaerythrit vernetzt sind,
- Copolymere von Acrylsäure und Methacrylsäure und Tetracosylacrylat, welche mit Allylethern von Sucrose vernetzt sind,
- Copolymere von Acrylsäure und Methacrylsäure und Tetracosylacrylat, welche mit Allylethern von Pentaerythrit vernetzt sind,
- Copolymere von Acrylsäure und Methacrylsäure und Pentacosylacrylat, welche mit Allylethern von Sucrose vernetzt sind,
- Copolymere von Acrylsäure und Methacrylsäure und Pentacosylacrylat, welche mit Allylethern von Pentaerythrit vernetzt sind,
- Copolymere von Acrylsäure und Methacrylsäure und Hexacosylacrylat, welche mit Allylethern von Sucrose vernetzt sind,
- Copolymere von Acrylsäure und Methacrylsäure und Hexacosylacrylat, welche mit Allylethern von Pentaerythrit vernetzt sind,
- Copolymere von Acrylsäure und Methacrylsäure und Heptacosylacrylat, welche mit Allylethern von Sucrose vernetzt sind,
- Copolymere von Acrylsäure und Methacrylsäure und Heptacosylacrylat, welche mit Allylethern von Pentaerythrit vernetzt sind,
- Copolymere von Acrylsäure und Methacrylsäure und Octacosylacrylat, welche mit Allylethern von Sucrose vernetzt sind,
- Copolymere von Acrylsäure und Methacrylsäure und Octacosylacrylat, welche mit Allylethern von Pentaerythrit vernetzt sind,
- Copolymere von Acrylsäure und Methacrylsäure und Nonacosylacrylat, welche mit Allylethern von Sucrose vernetzt sind,
- Copolymere von Acrylsäure und Methacrylsäure und Nonacosylacrylat, welche mit Allylethern von Pentaerythrit vernetzt sind,
- Copolymere von Acrylsäure und Methacrylsäure und Triacontylacrylat, welche mit Allylethern von Sucrose vernetzt sind,
- Copolymere von Acrylsäure und Methacrylsäure und Triacontylacrylat, welche mit Allylethern von Pentaerythrit vernetzt sind,
- Copolymere von Acrylsäure und Decylacrylat, welche mit Allylethern von Sucrose vernetzt sind,
- Copolymere von Acrylsäureestern und Decylacrylat, welche mit Allylethern von Pentaerythrit vernetzt sind,
- Copolymere von Acrylsäureestern und Undecylacrylat, welche mit Allylethern von Sucrose vernetzt sind,
- Copolymere von Acrylsäureestern und Undecylacrylat, welche mit Allylethern von Pentaerythrit vernetzt sind,
- Copolymere von Acrylsäureestern und Dodecylacrylat, welche mit Allylethern von Sucrose vernetzt sind,
- Copolymere von Acrylsäureestern und Dodecylacrylat, welche mit Allylethern von Pentaerythrit vernetzt sind,
- Copolymere von Acrylsäureestern und Tridecylacrylat, welche mit Allylethern von Sucrose vernetzt sind,
- Copolymere von Acrylsäureestern und Tridecylacrylat, welche mit Allylethern von Pentaerythrit vernetzt sind,
- Copolymere von Acrylsäureestern und Tetradecylacrylat, welche mit Allylethern von Sucrose vernetzt sind,
- Copolymere von Acrylsäureestern und Tetradecylacrylat, welche mit Allylethern von Pentaerythrit vernetzt sind,
- Copolymere von Acrylsäureestern und Pentadecylacrylat, welche mit Allylethern von Sucrose vernetzt sind,
- Copolymere von Acrylsäureestern und Pentadecylacrylat, welche mit Allylethern von Pentaerythrit vernetzt sind,
- Copolymere von Acrylsäureestern und Hexadecylacrylat, welche mit Allylethern von Sucrose vernetzt sind,
- Copolymere von Acrylsäureestern und Hexadecylacrylat, welche mit Allylethern von Pentaerythrit vernetzt sind,
- Copolymere von Acrylsäureestern und Heptadecylacrylat, welche mit Allylethern von Sucrose vernetzt sind,
- Copolymere von Acrylsäureestern und Heptadecylacrylat, welche mit Allylethern von Pentaerythrit vernetzt sind,
- Copolymere von Acrylsäureestern und Octadecylacrylat, welche mit Allylethern von Sucrose vernetzt sind,
- Copolymere von Acrylsäureestern und Octadecylacrylat, welche mit Allylethern von Pentaerythrit vernetzt sind,
- Copolymere von Acrylsäureestern und Nonadecylacrylat, welche mit Allylethern von Sucrose vernetzt sind,
- Copolymere von Acrylsäureestern und Nonadecylacrylat, welche mit Allylethern von Pentaerythrit vernetzt sind,
- Copolymere von Acrylsäureestern und Eicosylacrylat, welche mit Allylethern von Sucrose vernetzt sind,
- Copolymere von Acrylsäureestern und Eicosylacrylat, welche mit Allylethern von Pentaerythrit vernetzt sind,
- Copolymere von Acrylsäureestern und Heneicosylacrylat, welche mit Allylethern von Sucrose vernetzt sind,
- Copolymere von Acrylsäureestern und Heneicosylacrylat, welche mit Allylethern von Pentaerythrit vernetzt sind,
- Copolymere von Acrylsäureestern und Docosylacrylat, welche mit Allylethern von Sucrose vernetzt sind,
- Copolymere von Acrylsäureestern und Docosylacrylat, welche mit Allylethern von Pentaerythrit vernetzt sind,
- Copolymere von Acrylsäureestern und Tricosylacrylat, welche mit Allylethern von Sucrose vernetzt sind,
- Copolymere von Acrylsäureestern und Tricosylacrylat, welche mit Allylethern von Pentaerythrit vernetzt sind,
- Copolymere von Acrylsäureestern und Tetracosylacrylat, welche mit Allylethern von Sucrose vernetzt sind,
- Copolymere von Acrylsäureestern und Tetracosylacrylat, welche mit Allylethern von Pentaerythrit vernetzt sind,
- Copolymere von Acrylsäureestern und Pentacosylacrylat, welche mit Allylethern von Sucrose vernetzt sind,
- Copolymere von Acrylsäureestern und Pentacosylacrylat, welche mit Allylethern von Pentaerythrit vernetzt sind,
- Copolymere von Acrylsäureestern und Hexacosylacrylat, welche mit Allylethern von Sucrose vernetzt sind,
- Copolymere von Acrylsäureestern und Hexacosylacrylat, welche mit Allylethern von Pentaerythrit vernetzt sind,
- Copolymere von Acrylsäureestern und Heptacosylacrylat, welche mit Allylethern von Sucrose vernetzt sind,
- Copolymere von Acrylsäureestern und Heptacosylacrylat, welche mit Allylethern von Pentaerythrit vernetzt sind,
- Copolymere von Acrylsäureestern und Octacosylacrylat, welche mit Allylethern von Sucrose vernetzt sind,
- Copolymere von Acrylsäureestern und Octacosylacrylat, welche mit Allylethern von Pentaerythrit vernetzt sind,
- Copolymere von Acrylsäureestern und Nonacosylacrylat, welche mit Allylethern von Sucrose vernetzt sind,
- Copolymere von Acrylsäureestern und Nonacosylacrylat, welche mit Allylethern von Pentaerythrit vernetzt sind,
- Copolymere von Acrylsäureestern und Triacontylacrylat, welche mit Allylethern von Sucrose vernetzt sind,
- Copolymere von Acrylsäureestern und Triacontylacrylat, welche mit Allylethern von Pentaerythrit vernetzt sind,
- Copolymere von Methacrylsäureestern und Decylacrylat, welche mit Allylethern von Sucrose vernetzt sind,
- Copolymere von Methacrylsäureestern und Decylacrylat, welche mit Allylethern von Pentaerythrit vernetzt sind,
- Copolymere von Methacrylsäureestern und Undecylacrylat, welche mit Allylethern von Sucrose vernetzt sind,
- Copolymere von Methacrylsäureestern und Undecylacrylat, welche mit Allylethern von Pentaerythrit vernetzt sind,
- Copolymere von Methacrylsäureestern und Dodecylacrylat, welche mit Allylethern von Sucrose vernetzt sind,
- Copolymere von Methacrylsäureestern und Dodecylacrylat, welche mit Allylethern von Pentaerythrit vernetzt sind,
- Copolymere von Methacrylsäureestern und Tridecylacrylat, welche mit Allylethern von Sucrose vernetzt sind,
- Copolymere von Methacrylsäureestern und Tridecylacrylat, welche mit Allylethern von Pentaerythrit vernetzt sind,
- Copolymere von Methacrylsäureestern und Tetradecylacrylat, welche mit Allylethern von Sucrose vernetzt sind,
- Copolymere von Methacrylsäureestern und Tetradecylacrylat, welche mit Allylethern von Pentaerythrit vernetzt sind,
- Copolymere von Methacrylsäureestern und Pentadecylacrylat, welche mit Allylethern von Sucrose vernetzt sind,
- Copolymere von Methacrylsäureestern und Pentadecylacrylat, welche mit Allylethern von Pentaerythrit vernetzt sind,
- Copolymere von Methacrylsäureestern und Hexadecylacrylat, welche mit Allylethern von Sucrose vernetzt sind,
- Copolymere von Methacrylsäureestern und Hexadecylacrylat, welche mit Allylethern von Pentaerythrit vernetzt sind,
- Copolymere von Methacrylsäureestern und Heptadecylacrylat, welche mit Allylethern von Sucrose vernetzt sind,
- Copolymere von Methacrylsäureestern und Heptadecylacrylat, welche mit Allylethern von Pentaerythrit vernetzt sind,
- Copolymere von Methacrylsäureestern und Octadecylacrylat, welche mit Allylethern von Sucrose vernetzt sind,
- Copolymere von Methacrylsäureestern und Octadecylacrylat, welche mit Allylethern von Pentaerythrit vernetzt sind,
- Copolymere von Methacrylsäureestern und Nonadecylacrylat, welche mit Allylethern von Sucrose vernetzt sind,
- Copolymere von Methacrylsäureestern und Nonadecylacrylat, welche mit Allylethern von Pentaerythrit vernetzt sind,
- Copolymere von Methacrylsäureestern und Eicosylacrylat, welche mit Allylethern von Sucrose vernetzt sind,
- Copolymere von Methacrylsäureestern und Eicosylacrylat, welche mit Allylethern von Pentaerythrit vernetzt sind,
- Copolymere von Methacrylsäureestern und Heneicosylacrylat, welche mit Allylethern von Sucrose vernetzt sind,
- Copolymere von Methacrylsäureestern und Heneicosylacrylat, welche mit Allylethern von Pentaerythrit vernetzt sind,
- Copolymere von Methacrylsäureestern und Docosylacrylat, welche mit Allylethern von Sucrose vernetzt sind,
- Copolymere von Methacrylsäureestern und Docosylacrylat, welche mit Allylethern von Pentaerythrit vernetzt sind,
- Copolymere von Methacrylsäureestern und Tricosylacrylat, welche mit Allylethern von Sucrose vernetzt sind,
- Copolymere von Methacrylsäureestern und Tricosylacrylat, welche mit Allylethern von Pentaerythrit vernetzt sind,
- Copolymere von Methacrylsäureestern und Tetracosylacrylat, welche mit Allylethern von Sucrose vernetzt sind,
- Copolymere von Methacrylsäureestern und Tetracosylacrylat, welche mit Allylethern von Pentaerythrit vernetzt sind,
- Copolymere von Methacrylsäureestern und Pentacosylacrylat, welche mit Allylethern von Sucrose vernetzt sind,
- Copolymere von Methacrylsäureestern und Pentacosylacrylat, welche mit Allylethern von Pentaerythrit vernetzt sind,
- Copolymere von Methacrylsäureestern und Hexacosylacrylat, welche mit Allylethern von Sucrose vernetzt sind,
- Copolymere von Methacrylsäureestern und Hexacosylacrylat, welche mit Allylethern von Pentaerythrit vernetzt sind,
- Copolymere von Methacrylsäureestern und Heptacosylacrylat, welche mit Allylethern von Sucrose vernetzt sind,
- Copolymere von Methacrylsäureestern und Heptacosylacrylat, welche mit Allylethern von Pentaerythrit vernetzt sind,
- Copolymere von Methacrylsäureestern und Octacosylacrylat, welche mit Allylethern von Sucrose vernetzt sind,
- Copolymere von Methacrylsäureestern und Octacosylacrylat, welche mit Allylethern von Pentaerythrit vernetzt sind,
- Copolymere von Methacrylsäureestern und Nonacosylacrylat, welche mit Allylethern von Sucrose vernetzt sind,
- Copolymere von Methacrylsäureestern und Nonacosylacrylat, welche mit Allylethern von Pentaerythrit vernetzt sind,
- Copolymere von Methacrylsäureestern und Triacontylacrylat, welche mit Allylethern von Sucrose vernetzt sind,
- Copolymere von Methacrylsäureestern und Triacontylacrylat, welche mit Allylethern von Pentaerythrit vernetzt sind,
- Copolymere von Acrylsäure und Methacrylsäureestern und Decylacrylat, welche mit Allylethern von Sucrose vernetzt sind,
- Copolymere von Acrylsäure und Methacrylsäureestern und Decylacrylat, welche mit Allylethern von Pentaerythrit vernetzt sind,
- Copolymere von Acrylsäure und Methacrylsäureestern und Undecylacrylat, welche mit Allylethern von Sucrose vernetzt sind,
- Copolymere von Acrylsäure und Methacrylsäureestern und Undecylacrylat, welche mit Allylethern von Pentaerythrit vernetzt sind,
- Copolymere von Acrylsäure und Methacrylsäureestern und Dodecylacrylat, welche mit Allylethern von Sucrose vernetzt sind,
- Copolymere von Acrylsäure und Methacrylsäureestern und Dodecylacrylat, welche mit Allylethern von Pentaerythrit vernetzt sind,
- Copolymere von Acrylsäure und Methacrylsäureestern und Tridecylacrylat, welche mit Allylethern von Sucrose vernetzt sind,
- Copolymere von Acrylsäure und Methacrylsäureestern und Tridecylacrylat, welche mit Allylethern von Pentaerythrit vernetzt sind,
- Copolymere von Acrylsäure und Methacrylsäureestern und Tetradecylacrylat, welche mit Allylethern von Sucrose vernetzt sind,
- Copolymere von Acrylsäure und Methacrylsäureestern und Tetradecylacrylat, welche mit Allylethern von Pentaerythrit vernetzt sind,
- Copolymere von Acrylsäure und Methacrylsäureestern und Pentadecylacrylat, welche mit Allylethern von Sucrose vernetzt sind,
- Copolymere von Acrylsäure und Methacrylsäureestern und Pentadecylacrylat, welche mit Allylethern von Pentaerythrit vernetzt sind,
- Copolymere von Acrylsäure und Methacrylsäureestern und Hexadecylacrylat, welche mit Allylethern von Sucrose vernetzt sind,
- Copolymere von Acrylsäure und Methacrylsäureestern und Hexadecylacrylat, welche mit Allylethern von Pentaerythrit vernetzt sind,
- Copolymere von Acrylsäure und Methacrylsäureestern und Heptadecylacrylat, welche mit Allylethern von Sucrose vernetzt sind,
- Copolymere von Acrylsäure und Methacrylsäureestern und Heptadecylacrylat, welche mit Allylethern von Pentaerythrit vernetzt sind,
- Copolymere von Acrylsäure und Methacrylsäureestern und Octadecylacrylat, welche mit Allylethern von Sucrose vernetzt sind,
- Copolymere von Acrylsäure und Methacrylsäureestern und Octadecylacrylat, welche mit Allylethern von Pentaerythrit vernetzt sind,
- Copolymere von Acrylsäure und Methacrylsäureestern und Nonadecylacrylat, welche mit Allylethern von Sucrose vernetzt sind,
- Copolymere von Acrylsäure und Methacrylsäureestern und Nonadecylacrylat, welche mit Allylethern von Pentaerythrit vernetzt sind,
- Copolymere von Acrylsäure und Methacrylsäureestern und Eicosylacrylat, welche mit Allylethern von Sucrose vernetzt sind,
- Copolymere von Acrylsäure und Methacrylsäureestern und Eicosylacrylat, welche mit Allylethern von Pentaerythrit vernetzt sind,
- Copolymere von Acrylsäure und Methacrylsäureestern und Heneicosylacrylat, welche mit Allylethern von Sucrose vernetzt sind,
- Copolymere von Acrylsäure und Methacrylsäureestern und Heneicosylacrylat, welche mit Allylethern von Pentaerythrit vernetzt sind,
- Copolymere von Acrylsäure und Methacrylsäureestern und Docosylacrylat, welche mit Allylethern von Sucrose vernetzt sind,
- Copolymere von Acrylsäure und Methacrylsäureestern und Docosylacrylat, welche mit Allylethern von Pentaerythrit vernetzt sind,
- Copolymere von Acrylsäure und Methacrylsäureestern und Tricosylacrylat, welche mit Allylethern von Sucrose vernetzt sind,
- Copolymere von Acrylsäure und Methacrylsäureestern und Tricosylacrylat, welche mit Allylethern von Pentaerythrit vernetzt sind,
- Copolymere von Acrylsäure und Methacrylsäureestern und Tetracosylacrylat, welche mit Allylethern von Sucrose vernetzt sind,
- Copolymere von Acrylsäure und Methacrylsäureestern und Tetracosylacrylat, welche mit Allylethern von Pentaerythrit vernetzt sind,
- Copolymere von Acrylsäure und Methacrylsäureestern und Pentacosylacrylat, welche mit Allylethern von Sucrose vernetzt sind,
- Copolymere von Acrylsäure und Methacrylsäureestern und Pentacosylacrylat, welche mit Allylethern von Pentaerythrit vernetzt sind,
- Copolymere von Acrylsäure und Methacrylsäureestern und Hexacosylacrylat, welche mit Allylethern von Sucrose vernetzt sind,
- Copolymere von Acrylsäure und Methacrylsäureestern und Hexacosylacrylat, welche mit Allylethern von Pentaerythrit vernetzt sind,
- Copolymere von Acrylsäure und Methacrylsäureestern und Heptacosylacrylat, welche mit Allylethern von Sucrose vernetzt sind,
- Copolymere von Acrylsäure und Methacrylsäureestern und Heptacosylacrylat, welche mit Allylethern von Pentaerythrit vernetzt sind,
- Copolymere von Acrylsäure und Methacrylsäureestern und Octacosylacrylat, welche mit Allylethern von Sucrose vernetzt sind,
- Copolymere von Acrylsäure und Methacrylsäureestern und Octacosylacrylat, welche mit Allylethern von Pentaerythrit vernetzt sind,
- Copolymere von Acrylsäure und Methacrylsäureestern und Nonacosylacrylat, welche mit Allylethern von Sucrose vernetzt sind,
- Copolymere von Acrylsäure und Methacrylsäureestern und Nonacosylacrylat, welche mit Allylethern von Pentaerythrit vernetzt sind,
- Copolymere von Acrylsäure und Methacrylsäureestern und Triacontylacrylat, welche mit Allylethern von Sucrose vernetzt sind,
- Copolymere von Acrylsäure und Methacrylsäureestern und Triacontylacrylat, welche mit Allylethern von Pentaerythrit vernetzt sind,
- Copolymere von Acrylsäureestern und Methacrylsäure und Decylacrylat, welche mit Allylethern von Sucrose vernetzt sind,
- Copolymere von Acrylsäureestern und Methacrylsäure und Decylacrylat, welche mit Allylethern von Pentaerythrit vernetzt sind,
- Copolymere von Acrylsäureestern und Methacrylsäure und Undecylacrylat, welche mit Allylethern von Sucrose vernetzt sind,
- Copolymere von Acrylsäureestern und Methacrylsäure und Undecylacrylat, welche mit Allylethern von Pentaerythrit vernetzt sind,
- Copolymere von Acrylsäureestern und Methacrylsäure und Dodecylacrylat, welche mit Allylethern von Sucrose vernetzt sind,
- Copolymere von Acrylsäureestern und Methacrylsäure und Dodecylacrylat, welche mit Allylethern von Pentaerythrit vernetzt sind,
- Copolymere von Acrylsäureestern und Methacrylsäure und Tridecylacrylat, welche mit Allylethern von Sucrose vernetzt sind,
- Copolymere von Acrylsäureestern und Methacrylsäure und Tridecylacrylat, welche mit Allylethern von Pentaerythrit vernetzt sind,
- Copolymere von Acrylsäureestern und Methacrylsäure und Tetradecylacrylat, welche mit Allylethern von Sucrose vernetzt sind,
- Copolymere von Acrylsäureestern und Methacrylsäure und Tetradecylacrylat, welche mit Allylethern von Pentaerythrit vernetzt sind,
- Copolymere von Acrylsäureestern und Methacrylsäure und Pentadecylacrylat, welche mit Allylethern von Sucrose vernetzt sind,
- Copolymere von Acrylsäureestern und Methacrylsäure und Pentadecylacrylat, welche mit Allylethern von Pentaerythrit vernetzt sind,
- Copolymere von Acrylsäureestern und Methacrylsäure und Hexadecylacrylat, welche mit Allylethern von Sucrose vernetzt sind,
- Copolymere von Acrylsäureestern und Methacrylsäure und Hexadecylacrylat, welche mit Allylethern von Pentaerythrit vernetzt sind,
- Copolymere von Acrylsäureestern und Methacrylsäure und Heptadecylacrylat, welche mit Allylethern von Sucrose vernetzt sind,
- Copolymere von Acrylsäureestern und Methacrylsäure und Heptadecylacrylat, welche mit Allylethern von Pentaerythrit vernetzt sind,
- Copolymere von Acrylsäureestern und Methacrylsäure und Octadecylacrylat, welche mit Allylethern von Sucrose vernetzt sind,
- Copolymere von Acrylsäureestern und Methacrylsäure und Octadecylacrylat, welche mit Allylethern von Pentaerythrit vernetzt sind,
- Copolymere von Acrylsäureestern und Methacrylsäure und Nonadecylacrylat, welche mit Allylethern von Sucrose vernetzt sind,
- Copolymere von Acrylsäureestern und Methacrylsäure und Nonadecylacrylat, welche mit Allylethern von Pentaerythrit vernetzt sind,
- Copolymere von Acrylsäureestern und Methacrylsäure und Eicosylacrylat, welche mit Allylethern von Sucrose vernetzt sind,
- Copolymere von Acrylsäureestern und Methacrylsäure und Eicosylacrylat, welche mit Allylethern von Pentaerythrit vernetzt sind,
- Copolymere von Acrylsäureestern und Methacrylsäure und Heneicosylacrylat, welche mit Allylethern von Sucrose vernetzt sind,
- Copolymere von Acrylsäureestern und Methacrylsäure und Heneicosylacrylat, welche mit Allylethern von Pentaerythrit vernetzt sind,
- Copolymere von Acrylsäureestern und Methacrylsäure und Docosylacrylat, welche mit Allylethern von Sucrose vernetzt sind,
- Copolymere von Acrylsäureestern und Methacrylsäure und Docosylacrylat, welche mit Allylethern von Pentaerythrit vernetzt sind,
- Copolymere von Acrylsäureestern und Methacrylsäure und Tricosylacrylat, welche mit Allylethern von Sucrose vernetzt sind,
- Copolymere von Acrylsäureestern und Methacrylsäure und Tricosylacrylat, welche mit Allylethern von Pentaerythrit vernetzt sind,
- Copolymere von Acrylsäureestern und Methacrylsäure und Tetracosylacrylat, welche mit Allylethern von Sucrose vernetzt sind,
- Copolymere von Acrylsäureestern und Methacrylsäure und Tetracosylacrylat, welche mit Allylethern von Pentaerythrit vernetzt sind,
- Copolymere von Acrylsäureestern und Methacrylsäure und Pentacosylacrylat, welche mit Allylethern von Sucrose vernetzt sind,
- Copolymere von Acrylsäureestern und Methacrylsäure und Pentacosylacrylat, welche mit Allylethern von Pentaerythrit vernetzt sind,
- Copolymere von Acrylsäureestern und Methacrylsäure und Hexacosylacrylat, welche mit Allylethern von Sucrose vernetzt sind,
- Copolymere von Acrylsäureestern und Methacrylsäure und Hexacosylacrylat, welche mit Allylethern von Pentaerythrit vernetzt sind,
- Copolymere von Acrylsäureestern und Methacrylsäure und Heptacosylacrylat, welche mit Allylethern von Sucrose vernetzt sind,
- Copolymere von Acrylsäureestern und Methacrylsäure und Heptacosylacrylat, welche mit Allylethern von Pentaerythrit vernetzt sind,
- Copolymere von Acrylsäureestern und Methacrylsäure und Octacosylacrylat, welche mit Allylethern von Sucrose vernetzt sind,
- Copolymere von Acrylsäureestern und Methacrylsäure und Octacosylacrylat, welche mit Allylethern von Pentaerythrit vernetzt sind,
- Copolymere von Acrylsäureestern und Methacrylsäure und Nonacosylacrylat, welche mit Allylethern von Sucrose vernetzt sind,
- Copolymere von Acrylsäureestern und Methacrylsäure und Nonacosylacrylat, welche mit Allylethern von Pentaerythrit vernetzt sind,
- Copolymere von Acrylsäureestern und Methacrylsäure und Triacontylacrylat, welche mit Allylethern von Sucrose vernetzt sind,
- Copolymere von Acrylsäureestern und Methacrylsäure und Triacontylacrylat, welche mit Allylethern von Pentaerythrit vernetzt sind,
- Copolymere von Acrylsäureestern und Methacrylsäureestern und Decylacrylat, welche mit Allylethern von Sucrose vernetzt sind,
- Copolymere von Acrylsäureestern und Methacrylsäureestern und Decylacrylat, welche mit Allylethern von Pentaerythrit vernetzt sind,
- Copolymere von Acrylsäureestern und Methacrylsäureestern und Undecylacrylat, welche mit Allylethern von Sucrose vernetzt sind,
- Copolymere von Acrylsäureestern und Methacrylsäureestern und Undecylacrylat, welche mit Allylethern von Pentaerythrit vernetzt sind,
- Copolymere von Acrylsäureestern und Methacrylsäureestern und Dodecylacrylat, welche mit Allylethern von Sucrose vernetzt sind,
- Copolymere von Acrylsäureestern und Methacrylsäureestern und Dodecylacrylat, welche mit Allylethern von Pentaerythrit vernetzt sind,
- Copolymere von Acrylsäureestern und Methacrylsäureestern und Tridecylacrylat, welche mit Allylethern von Sucrose vernetzt sind,
- Copolymere von Acrylsäureestern und Methacrylsäureestern und Tridecylacrylat, welche mit Allylethern von Pentaerythrit vernetzt sind,
- Copolymere von Acrylsäureestern und Methacrylsäureestern und Tetradecylacrylat, welche mit Allylethern von Sucrose vernetzt sind,
- Copolymere von Acrylsäureestern und Methacrylsäureestern und Tetradecylacrylat, welche mit Allylethern von Pentaerythrit vernetzt sind,
- Copolymere von Acrylsäureestern und Methacrylsäureestern und Pentadecylacrylat, welche mit Allylethern von Sucrose vernetzt sind,
- Copolymere von Acrylsäureestern und Methacrylsäureestern und Pentadecylacrylat, welche mit Allylethern von Pentaerythrit vernetzt sind,
- Copolymere von Acrylsäureestern und Methacrylsäureestern und Hexadecylacrylat, welche mit Allylethern von Sucrose vernetzt sind,
- Copolymere von Acrylsäureestern und Methacrylsäureestern und Hexadecylacrylat, welche mit Allylethern von Pentaerythrit vernetzt sind,
- Copolymere von Acrylsäureestern und Methacrylsäureestern und Heptadecylacrylat, welche mit Allylethern von Sucrose vernetzt sind,
- Copolymere von Acrylsäureestern und Methacrylsäureestern und Heptadecylacrylat, welche mit Allylethern von Pentaerythrit vernetzt sind,
- Copolymere von Acrylsäureestern und Methacrylsäureestern und Octadecylacrylat, welche mit Allylethern von Sucrose vernetzt sind,
- Copolymere von Acrylsäureestern und Methacrylsäureestern und Octadecylacrylat, welche mit Allylethern von Pentaerythrit vernetzt sind,
- Copolymere von Acrylsäureestern und Methacrylsäureestern und Nonadecylacrylat, welche mit Allylethern von Sucrose vernetzt sind,
- Copolymere von Acrylsäureestern und Methacrylsäureestern und Nonadecylacrylat, welche mit Allylethern von Pentaerythrit vernetzt sind,
- Copolymere von Acrylsäureestern und Methacrylsäureestern und Eicosylacrylat, welche mit Allylethern von Sucrose vernetzt sind,
- Copolymere von Acrylsäureestern und Methacrylsäureestern und Eicosylacrylat, welche mit Allylethern von Pentaerythrit vernetzt sind,
- Copolymere von Acrylsäureestern und Methacrylsäureestern und Heneicosylacrylat, welche mit Allylethern von Sucrose vernetzt sind,
- Copolymere von Acrylsäureestern und Methacrylsäureestern und Heneicosylacrylat, welche mit Allylethern von Pentaerythrit vernetzt sind,
- Copolymere von Acrylsäureestern und Methacrylsäureestern und Docosylacrylat, welche mit Allylethern von Sucrose vernetzt sind,
- Copolymere von Acrylsäureestern und Methacrylsäureestern und Docosylacrylat, welche mit Allylethern von Pentaerythrit vernetzt sind,
- Copolymere von Acrylsäureestern und Methacrylsäureestern und Tricosylacrylat, welche mit Allylethern von Sucrose vernetzt sind,
- Copolymere von Acrylsäureestern und Methacrylsäureestern und Tricosylacrylat, welche mit Allylethern von Pentaerythrit vernetzt sind,
- Copolymere von Acrylsäureestern und Methacrylsäureestern und Tetracosylacrylat, welche mit Allylethern von Sucrose vernetzt sind,
- Copolymere von Acrylsäureestern und Methacrylsäureestern und Tetracosylacrylat, welche mit Allylethern von Pentaerythrit vernetzt sind,
- Copolymere von Acrylsäureestern und Methacrylsäureestern und Pentacosylacrylat, welche mit Allylethern von Sucrose vernetzt sind,
- Copolymere von Acrylsäureestern und Methacrylsäureestern und Pentacosylacrylat, welche mit Allylethern von Pentaerythrit vernetzt sind,
- Copolymere von Acrylsäureestern und Methacrylsäureestern und Hexacosylacrylat, welche mit Allylethern von Sucrose vernetzt sind,
- Copolymere von Acrylsäureestern und Methacrylsäureestern und Hexacosylacrylat, welche mit Allylethern von Pentaerythrit vernetzt sind,
- Copolymere von Acrylsäureestern und Methacrylsäureestern und Heptacosylacrylat, welche mit Allylethern von Sucrose vernetzt sind,
- Copolymere von Acrylsäureestern und Methacrylsäureestern und Heptacosylacrylat, welche mit Allylethern von Pentaerythrit vernetzt sind,
- Copolymere von Acrylsäureestern und Methacrylsäureestern und Octacosylacrylat, welche mit Allylethern von Sucrose vernetzt sind,
- Copolymere von Acrylsäureestern und Methacrylsäureestern und Octacosylacrylat, welche mit Allylethern von Pentaerythrit vernetzt sind,
- Copolymere von Acrylsäureestern und Methacrylsäureestern und Nonacosylacrylat, welche mit Allylethern von Sucrose vernetzt sind,
- Copolymere von Acrylsäureestern und Methacrylsäureestern und Nonacosylacrylat, welche mit Allylethern von Pentaerythrit vernetzt sind,
- Copolymere von Acrylsäureestern und Methacrylsäureestern und Triacontylacrylat, welche mit Allylethern von Sucrose vernetzt sind,
- Copolymere von Acrylsäureestern und Methacrylsäureestern und Triacontylacrylat, welche mit Allylethern von Pentaerythrit vernetzt sind.

Diese Polymere oder Mischungen daraus sind ebenfalls im Handel erhältlich, beispielsweise unter den Bezeichnungen Carbopol® 1342, Pemulen® TR-1 und Pemulen® TR2.

Erfindungsgemäß bevorzugte Haarreinigungsmittel enthalten die Komponente(n) c) in Mengen von 0,2 bis 4 Gew.-%, vorzugsweise von 0,3 bis 3 Gew.-%, besonders bevorzugt von 0,4 bis 2,5 Gew.-% und insbesondere von 0,5 bis 1,5 Gew.-%, jeweils bezogen auf das gesamte Mittel.

Die erfindungsgemäßen Haarreinigungsmittel enthalten 5 bis 50 Gew.-% Tensid(e). Bevorzugte erfindungsgemäße Haarreinigungsmittel sind dadurch gekennzeichnet, daß sie - bezogen auf sein Gewicht - 5 bis 45 Gew.-%, vorzugsweise 6 bis 42 Gew.-% und insbesondere 7,5 bis 25 Gew.-% anionische(s) und/oder nichtionische(s) und/oder kationische(s) und/oder amphotere(s) Tensid(e), enthalten.

Unter dem Begriff Tenside werden grenzflächenaktive Substanzen, die an Ober- und Grenzflächen Adsorptionsschichten bilden oder in Volumenphasen zu Mizellkolloiden oder lyotropen Mesophasen aggregieren können, verstanden. Man unterscheidet Aniontenside bestehend aus einem hydrophoben Rest und einer negativ geladenen hydrophilen Kopfgruppe, amphotere Tenside, welche sowohl eine negative als auch eine kompensierende positive Ladung tragen, kationische Tenside, welche neben einem hydrophoben Rest eine positiv geladene hydrophile Gruppe aufweisen, und nichtionische Tenside, welche keine Ladungen sondern starke Dipolmomente aufweisen und in wäßriger Lösung stark hydratisiert sind.

Als anionische Tenside (E1) eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykoloder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
- sulfatierte Hydroxyalkylpolyethylen- und/oder Hydroxyalkylenpropylenglykolether
- Sulfonate ungesättigter Fettsäuren mit 8 bis 24 C-Atomen und 1 bis 6 Doppelbindungen
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (E1-I),

   R¹(OCH₂CH₂)ₙ-O-P(O)(OX)-OR² (E1-I)

   in der R¹ bevorzugt für einen aliphatischen Kohlenwasserstoffrest mit 8 bis 30 Kohlenstoffatomen, R² für Wasserstoff, einen Rest (CH₂CH₂O)ₙR² oder X, n für Zahlen von 1 bis 10 und X für Wasserstoff, ein Alkali- oder Erdalkalimetall oder NR³R⁴R⁵R⁶, mit R³ bis R⁶ unabhängig voneinander stehend für Wasserstoff oder einen C₁ bis C₄- Kohlenwasserstoffrest, steht,
- sulfatierte Fettsäurealkylenglykolester der Formel (E1-II)

   R⁷CO(AlkO)ₙSO₃M (E1-II)

   in der R⁷CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht,
- Monoglyceridsulfate und Monoglyceridethersulfate der Formel (E1-III) 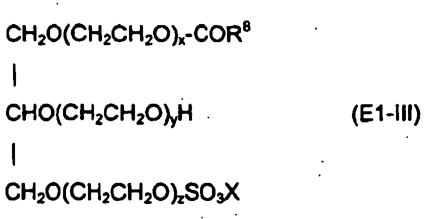 in der R⁸C0 für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate der Formel (E1-III) eingesetzt, in der R⁸CO für einen linearen Acylrest mit 8 bis 18 Kohlenstoffatomen steht, ,
- Amidethercarbonsäuren,
- Kondensationsprodukte aus C₈ - C₃₀ - Fettalkoholen mit Proteinhydrolysaten und/oder Aminosäuren und deren Derivaten, welche dem Fachmann als Eiweissfettsäurekondensate bekannt sind, wie beispielsweise die Lamepon® - Typen, Gluadin® - Typen, Hostapon® KCG oder die Amisoft® - Typen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen, Monoglycerdisulfate, Alkyl- und Alkenyletherphosphate sowie Eiweissfettsäurekondensate.

Als zwitterionische Tenside (E2) werden solche oberflächenaktiven Verbindungen bezeichnet, die im Molekül mindestens eine quartäre Ammoniumgruppe und mindestens eine -COO⁽⁻⁾ - oder -SO₃⁽⁻⁾ -Gruppe tragen. Besonders geeignete zwitterionische Tenside sind die sogenannten Betaine wie die N-Alkyl-N,N-dimethylammoniumglycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden (E3) werden solche oberflächenaktiven Verbindungen verstanden, die außer einer C₈ - C₂₄ - Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂-C₁₈- Acylsarcosin.

Nichtionische Tenside (E4) enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂ - C₆ - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol® LS, Dehydol® LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen® HSP (Cognis) oder Sovermol - Typen (Cognis),
- alkoxilierte Triglyceride,
- alkoxilierte Fettsäurealkylester der Formel (E4-I)

   R¹CO-(OCH₂CHR²)_{w}OR³ (E4-I)

   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
- Aminoxide,
- Hydroxymischether,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),

   R⁴O-[G]ₚ (E4-II)

   in der R⁴ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden.
   Die Alkyl- und Alkenyloligoglykoside können sich von Aldosen bzw. Ketosen mit 5 oder 6 Kohlenstoffatomen, vorzugsweise von Glucose, ableiten. Die bevorzugten Alkyl- und/oder Alkenyloligoglykoside sind somit Alkyl- und/oder Alkenyloligoglucoside. Die Indexzahl p in der allgemeinen Formel (E4-II) gibt den Oligomerisierungsgrad (DP), d. h. die Verteilung von Mono- und Oligoglykosiden an und steht für eine Zahl zwischen 1 und 10. Während p im einzelnen Molekül stets ganzzahlig sein muß und hier vor allem die Werte p = 1 bis 6 annehmen kann, ist der Wert p für ein bestimmtes Alkyloligoglykosid eine analytisch ermittelte rechnerische Größe; die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R⁴ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-0xoalkohole (DP = 1 bis 3). Der Alkyl- bzw. Alkenylrest R¹⁵ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.
- Zuckertenside vom Typ der Fettsäure-N-alkylpolyhydroxyalkylamide, ein nichtionisches Tensid der Formel (E4-III),

   R⁵CO-NR⁶-[Z] (E4-III)

   in der R⁵CO für einen aliphatischen Acylrest mit 6 bis 22 Kohlenstoffatomen, R⁶ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht. Bei den Fettsäure-N-alkylpolyhydroxyalkylamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden. Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher Fettsäure-N-alkylglucamide dar, wie sie durch die Formel (E4-IV) wiedergegeben werden:

   R⁷CO-NR⁸-CH₂-(CHOH)₄-CH₂OH (E4-IV)

   Vorzugsweise werden als Fettsäure-N-alkylpolyhydroxyalkylamide Glucamide der Formel (E4-IV) eingesetzt, in der R⁸ für Wasserstoff oder eine Alkylgruppe steht und R⁷CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. derer technischer Mischungen steht. Besonders bevorzugt sind Fettsäure-N-alkylglucamide der Formel (E4-IV), die durch reduktive Aminierung von Glucose mit Methylamin und anschließende Acylierung mit Laurinsäure oder C12/14-Kokosfettsäure bzw. einem entsprechenden Derivat erhalten werden. Weiterhin können sich die Polyhydroxyalkylamide auch von Maltose und Palatinose ableiten.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Diese Verbindungen sind durch die folgenden Parameter gekennzeichnet. Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Weiterhin sind ganz besonders bevorzugte nichtionische Tenside die Zuckertenside. Diese können in den erfindungsgemäß verwendeten Mitteln bevorzugt in Mengen von 0,1 - 20 Gew.-%, bezogen auf das gesamte Mittel, enthalten sein. Mengen von 0,5 - 15 Gew.-% sind bevorzugt, und ganz besonders bevorzugt sind Mengen von 0,5 - 7,5 Gew.%.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Erfindungsgemäß einsetzbar sind kationische Tenside vom Typ der quarternären Ammoniumverbindungen, der Esterquats und der Amidoamine. Bevorzugte quatemäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quatemium-83 bekannten Imidazolium-Verbindungen. Die langen Alkylketten der oben genannten Tenside weisen bevorzugt 10 bis 18 Kohlenstoffatome auf.

Bevorzugt einsetzbar sind erfindungsgemäß QAV mit Behenylresten, insbesondere die unter der Bezeichnung Behentrimoniumchlorid bzw. -bromid (Docosanyltrimethylammonium Chlorid bzw. -Bromid) bekannten Substanzen. Andere bevorzugte QAV weisen mindestens zwei Behenylreste auf, wobei QAV, welche zwei Behenylreste an einem Imidazoliniumrückgrat besonders bevorzugt sind. Kommerziell erhältlich sind diese Substanzen beispielsweise unter den Bezeichnungen Genamin® KDMP (Clariant) und Crodazosoft® DBQ (Crodauza).

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis(2-Palmitoyloxy- - ethyl)dimethylammoniumchlorid, sowie Dehyquart® F-75, Dehyquart® C-4046, Dehyquart® L80 und Dehyquart® AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfin-dungsgemäß besonders geeignete Verbindung aus dieser Substanzgruppe stellt das unter der Bezeichnung Tegoamid® S 18 im Handel erhältliche Stearamidopropyldimethylamin dar.

Die kationischen Tenside sind in den erfindungsgemäßen Mitteln bevorzugt in Mengen von 0,05 bis 10 Gew.-%, bezogen auf das gesamte Mittel, enthalten. Mengen von 0,1 bis 5 Gew.-% sind besonders bevorzugt. "Bezogen auf das gesamte Mittel" bedeutet dabei im rahmen der vorliegenden Erfindung "bezogen auf die Mischung der Zubereitung von Oxidationsfarbstoffvorprodukten (A) und der Oxidationsmittelzubereitung (B).

Anionische, nichtionische, zwitterionische und/oder amphotere Tenside sowie deren Mischungen können erfindungsgemäß bevorzugt sein.

Die erfindungsgemäßen Haarreinigungsmittel können Silikon(e) in Mengen von 0,05 bis 90 Gew.%, jeweils bezogen auf das gesamte Mittel enthalten. Dabei enthalten üblicherweise nur spezielle Produkte wie beispielsweise Haarspitzenfluids hohe Mengen an Silikonen, während andere Produkte wie Shampoos, Conditioner, Haarkuren usw. eher Mengen unterhalb 20 Gew.-% enthalten. Je nach Art des erfindungsgemäßen Mittels sind daher die Gehalte an Silikon(en) durchaus unterschiedlich. Werden die erfindungsgemäßen Mittel als Shampoos konfektioniert, sind besonders bevorzugte erfindungsgemäße Haarreinigungsmittel dadurch gekennzeichnet, daß sie Silikon(e) in Mengen von 0,1 bis 10 Gew.-%, vorzugsweise von 0,25 bis 5 Gew.-% und insbesondere von 0,5 bis 2,5 Gew.-%, jeweils bezogen auf das gesamte Mittel, enthalten.

Insbesondere bevorzugt sind erfindungsgemäße Haarreinigungsmittel, die mindestens ein Silicon enthalten, das ausgewählt ist unter:
(i) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;
(ii) Polysiloxanen, die in ihrer allgemeinen Struktur eine oder mehrere organofunktionelle Gruppen enthalten, die ausgewählt sind unter:
   a) substituierten oder unsubstituierten aminierten Gruppen;
   b) (per)fluorierten Gruppen;
   c) Thiolgruppen;
   d) Carboxylatgruppen;
   e) hydroxylierten Gruppen;
   f) alkoxylierten Gruppen;
   g) Acyloxyalkylgruppen;
   h) amphoteren Gruppen;
   i) Bisulfitgruppen;
   j) Hydroxyacylaminogruppen;
   k) Carboxygruppen;
   l) Sulfonsäuregruppen; und
   m) Sulfat- oder Thiosulfatgruppen;
(iii) linearen Polysiloxan(A)- Polyoxyalkylen(B)- Blockcopoylmeren vom Typ (AB)ₙ mit n > 3;
(iv) gepfropften Siliconpolymeren mit nicht siliconhaltigem, organischen Grundgerüst, die aus einer organischen Hauptkette bestehen, welche aus organischen Monomeren gebildet wird, die kein Silicon enthalten, auf die in der Kette sowie gegebenenfalls an mindestens einem Kettenende mindestens ein Polysiloxanmakromer gepfropft wurde;
(v) gepfropften Siliconpolymeren mit Polysiloxan- Grundgerüst, auf das nicht siliconhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silicon enthält;
(vi) oder deren Gemischen.

Besonders bevorzugte erfindungsgemäße Haarreinigungsmittel sind dadurch gekennzeichnet, daß sie zusätzlich mindestens ein Silikon der Formel 1

(CH₃)₃Si-[O-Si(CH₃)₂]ₓ-O-Si(CH₃)₃ (I),

enthalten, in der x für eine Zahl von 0 bis 100, vorzugsweise von 0 bis 50, weiter bevorzugt von 0 bis 20 und insbesondere 0 bis 10, steht.

Die erfindungsgemäß bevorzugten Haarreinigungsmittel enthalten ein Silikon der vorstehenden Formel I. Diese Silikone werden nach der INCI-Nomenklatur als DIMETHICONE bezeichnet. Es werden im Rahmen der vorliegenden Erfindung als Silicon der Formel I vorzugsweise die Verbindungen:

(CH₃)₃Si-O-Si(CH₃)₃

(CH₃)₃Si-O-(CH₃)₂Si-O-Si(CH₃)₃

(CH₃)₃Si-[O-(CH₃)₂Si]₂-O-Si(CH₃)₃

(CH₃)₃Si-[O-(CH₃)₂Si]₃-O-Si(CH₃)₃

(CH₃)₃Si-[O-(CH₃)₂Si]₄-O-Si(CH₃)₃

(CH₃)₃Si-[O-(CH₃)₂Si]₅-O-si(CH₃)₃

(CH₃)₃Si-[O-(CH₃)₂Si]₆-O-Si(CH₃)₃

(CH₃)₃Si-[O-(CH₃)₂Si]₇-O-Si(CH₃)₃

(CH₃)₃Si-[O-(CH₃)₂Si]₈-O-Si(CH₃)₃

(CH₃)₃Si-[O-(CH₃)₂Si]₉-O-Si(CH₃)₃

(CH₃)₃Si-[O-(CH₃)₂Si]₁₀-O-Si(CH₃)₃

(CH₃)₃Si-[O-(CH₃)₂Si]₁₁-O-Si(CH₃)₃

(CH₃)₃Si-[O-(CH₃)₂Si]₁₂-O-Si(CH₃)₃

(CH₃)₃Si-[O-(CH₃)₂Si]₁₃-O-Si(CH₃)₃

(CH₃)₃Si-[O-(CH₃)₂Si]₁₄-O-Si(CH₃)₃

(CH₃)₃Si-[O-(CH₃)₂Si]₁₅-O-Si(CH₃)₃

(CH₃)₃Si-[O-(CH₃)₂Si]₁₆-O-Si(CH₃)₃

(CH₃)₃Si-[O-(CH₃)₂Si]₁₇-O-Si(CH₃)₃

(CH₃)₃Si-[O-(CH₃)₂Si]₁₈-O-Si(CH₃)₃

(CH₃)₃Si-[O-(CH₃)₂Si]₁₉-O-Si(CH₃)₃

(CH₃)₃Si-[O-(CH₃)₂Si]₂₀-O-Si(CH₃)₃

eingesetzt, wobei (CH₃)₃Si-O-Si(CH₃)₃, (CH₃)₃Si-O-(CH₃)₂Si]₂-O-Si(CH₃)₃ und/oder (CH₃)₃Si-[O-(CH₃)₂Si]₂-O-Si(CH₃)₃ besonders bevorzugt sind.

Selbstverständlich können auch Mischungen der o.g. Silikone in den erfindungsgemäßen Mitteln enthalten sein.

Bevorzugte erfindungsgemäß einsetzbare Silikone weisen bei 20°C Viskositäten von 0,2 bis 2 mm²s⁻¹ auf, wobei Silikone mit Viskositäten von 0,5 bis 1 mm²s⁻¹ besonders bevorzugt sind.

Besonders bevorzugte erfindungsgemäße Mittel enthalten ein oder mehrere aminofunktionelle Silicone. Solche Silicone können z.B. durch die Formel

M(RₐQ_{b}SiO_{(4-a-b)/2)x}(R_{c}SiO_{(4-c)/2)y}M

Beschreiben werden, wobei in der obigen Formel R ein Kohlenwasserstoff oder ein Kohlenwasserstoffrest mit 1 bis etwa 6 Kohlenstoffatomen ist, Q ein polarer Rest der allgemeinen Formel -R¹HZ ist, worin R¹ eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und Z ein organischer, aminofunktioneller Rest ist, der mindestens eine aminofunktionelle Gruppe enthält; "a" Werte im Bereich von etwa 0 bis etwa 2 annimmt, "b" Werte im Bereich von etwa 1 bis etwa 3 annimmt, "a" + "b" kleiner als oder gleich 3 ist, und "c" eine Zahl im Bereich von etwa 1 bis etwa 3 ist, und x eine Zahl im Bereich von 1 bis etwa 2.000, vorzugsweise von etwa 3 bis etwa 50 und am bevorzugtesten von etwa 3 bis etwa 25 ist, und y eine Zahl im Bereich von etwa 20 bis etwa 10.000, vorzugsweise von etwa 125 bis etwa 10.000 und am bevorzugtesten von etwa 150 bis etwa 1.000 ist, und M eine geeignete Silicon-Endgruppe ist, wie sie im Stande der Technik bekannt ist, vorzugsweise Trimethylsiloxy. Nicht einschränkende Beispiele der durch R repräsentierten Reste schließen Alkylreste, wie Methyl, Ethyl, Propyl, Isopropyl, Isopropyl Butyl, Isobutyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3-Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R ein Alkylrest, der 1 bis etwa 6 Kohlenstoffatomen enthält, und am bevorzugtesten ist R Methyl. Beispiele von R¹ schließen Methylen, Ethylen, Propylen, Hexamethylen, Decamethylen, - CH₂CH(CH₃)CH₂-, Phenylen, Naphthylen, -CH₂CH₂SCH₂CH₂-, -CH₂CH₂OCH₂-, - OCH₂CH₂-, -OCH₂ CH₂CH₂-, -CH₂CH(CH₃)C(O)OCH₂-, -(CH₂)₃ CC(O)OCH₂CH₂-, -C₆H₄C₆H₄-, -C₆H₄CH₂C₆H₄-; und -(CH₂)₃C(O)SCH₂CH₂- ein.

Z ist ein organischer, aminofunktioneller Rest, enthaltend mindestens eine funktionelle Aminogruppe. Eine mögliche Formel für Z ist NH(CH₂)_{z}NH₂, worin z 1 oder mehr ist. Eine andere mögliche Formel für Z ist -NH(CH₂)_{z} (CH₂)_{zz}NH, worin sowohl z als auch zz unabhängig 1 oder mehr sind, wobei diese Struktur Diamino-Ringstrukturen umfaßt, wie Piperazinyl. Z ist am bevorzugtesten ein -NHCH₂CH₂NH₂-Rest. Eine andere mögliche Formel für Z ist - N(CH₂)_{z}(CH₂)_{zz}NX₂ oder -NX₂, worin jedes X von X₂ unabhängig ausgewählt ist aus der Gruppe bestehend aus Wasserstoff und Alkylgruppen mit 1 bis 12 Kohlenstoffatomen, und zz 0 ist.

Q ist am bevorzugtesten ein polarer, aminfunktioneller Rest der Formel - CH₂CH₂CH₂NHCH₂CH₂NH₂. In den Formeln nimmt "a" Werte im Bereich von etwa 0 bis etwa 2 an, "b" nimmt Werte im Bereich von etwa 2 bis etwa 3 an, "a" + "b" ist kleiner als oder gleich 3, und "c" ist eine Zahl im Bereich von etwa 1 bis etwa 3. Das molare Verhältnis der RₐQ_{b} SiO_{(4-a-b)/2}-Einheiten zu den R_{c}SiO_{(4-c)/2}-Einheiten liegt im Bereich von etwa 1 : 2 bis 1 : 65, vorzugsweise von etwa 1 : 5 bis etwa 1 : 65 und am bevorzugtesten von etwa 1 : 15 bis etwa 1 : 20. Werden ein oder mehrere Silicone der obigen Formel eingesetzt, dann können die verschiedenen variablen Substituenten in der obigen Formel bei den verschiedenen Siliconkomponenten, die in der Siliconmischung vorhanden sind, verschieden sein.

Bevorzugte erfindungsgemäße Haarreinigungsmittel sind dadurch gekennzeichnet, daß sie ein aminofunktionelles Silikon der Formel II

R'ₐG₃₋ₐ-Si(OSiG₂)ₙ-(OSiG_{b}R'_{2-b})ₘ-O-SiG₃₋ₐ-R'ₐ (II),

enthalten, worin bedeutet:
- G ist-H, eine Phenylgruppe, -OH, -O-CH₃, -CH₃, -O-CH₂CH₃, -CH₂CH₃, -O-CH₂CH₂CH₃, -CH₂CH₂CH₃, -O-CH(CH₃)₂,- -CH(CH₃)₂, -O-CH₂CH₂CH₂CH₃, -CH₂CH₂CH₂CH₃, -O-CH₂CH(CH₃)₂, -CH₂CH(CH₃)₂, -O-CH(CH₃)CH₂CH₃, -CH(CH₃)CH₂CH₃, -O-C(CH₃)₃, -C(CH₃)₃;
- a steht für eine Zahl zwischen 0 und 3, insbesondere 0;
- b steht für eine Zahl zwischen 0 und 1, insbesondere 1,
- m und n sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R' ist ein monovalenter Rest ausgewählt aus
   ∘ -Q-N(R")-CH₂-CH₂-N(R")₂
   ∘ -Q-N(R")₂
   ∘ -Q-N⁺(R")₃A⁻
   ∘ -Q-N⁺H(R")₂ A⁻
   ∘ -Q-N⁺H₂(R")A⁻
   ∘ -Q-N(R")-CH₂-CH₂-N⁺R"H₂A⁻,
   wobei jedes Q für eine chemische Bindung, -CH₂-, -CH₂-CH₂-, -CH₂CH₂CH₂-, -C(CH₃)₂-,-CH₂CH₂CH₂CH₂-, -CH₂C(CH₃)₂-, -CH(CH₃)CH₂CH₂- steht,
   R" für gleiche oder verschiedene Reste aus der Gruppe -H, -Phenyl, -Benzyl, -CH₂₋CH(CH₃)Ph, der C₁₋₂₀-Alkylreste, vorzugsweise -CH₃, -CH₂CH₃, -CH₂CH₂CH₃, -CH(CH₃)₂, -CH₂CH₂CH₂H₃, -CH₂CH(CH₃)₂, -CH(CH₃)CH₂CH₃, -C(CH₃)₃, steht und A ein Anion repräsentiert, welches vorzugsweise ausgewählt ist aus Chlorid, Bromid, Iodid oder Methosulfat.

Besonders bevorzugte erfindungsgemäße Haarreinigungsmittel sind dadurch gekennzeichnet, daß sie mindestens es ein aminofunktionelles Silikon der Formel (IIa) 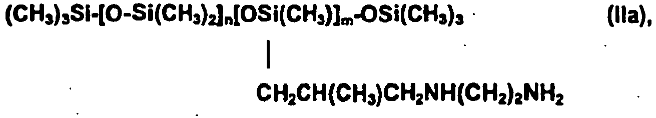 enthalten, worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Diese Silicone werden nach der INCI-Deklaration als Trimethylsilylamodimethicone bezeichnet.

Besonders bevorzugt sind auch erfindungsgemäße Haarreinigungsmittel, die mindestens ein aminofunktionelles Silikon der Formel (IIb) 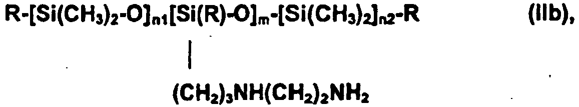 enthalten, worin R für -OH, -O-CH₃ oder eine -CH₃-Gruppe steht und m, n1 und n2 Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese Silicone werden nach der INCI-Deklaration als Amodimethicone bezeichnet.

Unabhängig davon, welche aminofunktionellen Silicone eingesetzt werden, sind erfindungsgemäße Haarreinigungsmittel bevorzugt, die ein aminofunktionelles Silikon enthalten dessen Aminzahl oberhalb von 0,25 meq/g, vorzugsweise oberhalb von 0,3 meq/g und insbesondere oberhalb von 0,4 meq/g liegt. Die Aminzahl steht dabei für die Milli-Äquivalente Amin pro Gramm des aminofunktionellen Silicons. Sie kann durch Titration ermittelt und auch in der Einheit mg KOH/g angegeben werden.

Erfindungsgemäß bevorzugte Haarreinigungsmittel sind dadurch gekennzeichnet, daß sie, bezogen auf ihr Gewicht, 0,01 bis 10 Gew.%, vorzugsweise 0,1 bis 8 Gew.%, besonders bevorzugt 0,25 bis 7,5 Gew.% und insbesondere 0,5 bis 5 Gew.% aminofunktionelle(s) Silikon(e) enthalten.

Auch die nach INCI als CYCLOMETHICONE bezeichneten cyclischen Dimethicone sind erfindungsgemäß mit Vorzug einsetzbar. Hier sind erfindungsgemäße Haarreinigungsmittel bevorzugt, die mindestens ein Silikon der Formel III 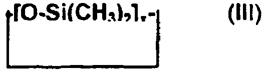 enthalten, in der x für eine Zahl von 0 bis 200, vorzugsweise von 0 bis 10, weiter bevorzugt von 0 bis 7 und insbesondere 0, 1, 2, 3, 4, 5 oder 6, steht.

Die vorstehend beschriebenen Silicone weisen ein Rückgrat auf, welches aus -Si-O-Si-Einheiten aufgebaut ist. Selbstverständlich können diese Si-O-Si-Einheiten auch durch Kohlenstoffketten unterbrochen sein. Entsprechende Moleküle sind durch Kettenverlängerungsreaktionen zugänglich und kommen vorzugsweise in Form von Silikon-in-Wasser-Emulsionen zum Einsatz.

Die erfindungsgemäß einsetzbaren Silikon-in-Wasser Emulsionen können nach bekannten Verfahren hergestellt werden, wie sie beispielsweise in US 5,998,537 und EP 0 874 017 A1 offenbart sind.

Zusammenfassend umfaßt dieses Herstellungsverfahren die emulgierende Mischung von Komponenten, deren eine mindestens ein Polysiloxane enthält, deren andere mindestens ein Organosilikonmaterial enthält, das mit dem Polysiloxane in einer Kettenverlängerungsreaktion reagiert, wobei mindestens ein ein Metallion-enthaltender Katalysator für die Kettenverlängerungsreaktion, mindestens ein Tensid und Wasser zugegen sind.

Kettenverlängerungsreaktionen mit Polysiloxanen sind bekannt und können beispielsweise die Hydrosüylierungsreaktion umfassen, in der eine Si-H Gruppe mit einer aliphatisch ungesättigten Gruppe in Gegenwart eines Platin/Rhodium-Katalysators unter Bildung von Polysiloxanes mit einigen Si-(C)ₚ-Si Bindungen (p = 1-6) reagiert, wobei die Polysiloxane auch als Polysiloxane-Polysilalkylene-Copolymere bezeichnet werden.

Die Kettenverlängerungsreaktion kann auch die Reaktion einer Si-OH Gruppe (beispielsweise eines Hydroxy-terminierten Polysiloxans) mit einer Alkoxygruppe (beispielsweise Alkoxysilanen, Silikaten oder Alkoxysiloxanen) in Gegenwart eines metallhaltigen Katalysators unter Bildung von Polysiloxanen umfassen.

Die Polysiloxane, die in der Kettenverlängerungsreaktion eingesetzt werden, umfassen ein substantiell lineares Polymer der folgenden Struktur:

R-Si(R₂)-[-O-Si(R₂)-]ₙ-O-SiR₃

In dieser Struktur steht jedes R unabhängig voneinander für einen Kohlenwasserstoffrest mit bis zu 20 Kohlenstoffatomen, vorzugsweise mit 1 bis 6 C-Atomen, wie beispielsweise einer Alkylgruppe (beispielsweise Methyl, Ethyl, Propyl oder Butyl), eine Arylgruppe (beispielsweise Phenyl), oder die für die Kettenverlängerungsreaktion benötigte Gruppe ("reaktive Gruppe", beispielsweise Si-gebundene H-Atome, aliphatisch ungesättigte Gruppen wie Vinyl, Allyl oder Hexenyl, Hydroxy, Alkoxy wie Methoxy, Ethoxy oder Propoxy, Alkoxy-Alkoxy, Acetoxy, Amino usw.), mit der Maßgabe, daß durchschnittlich ein bis zwei reaktive Gruppen pro Polymer vorliegen, n ist eine positive Zahl > 1. Vorzugsweise ist eine Mehrzahl der reaktiven Gruppen, besonders bevorzugt > 90%, und insbesondere > 98% der reaktiven Gruppen, an den endständigen Si-Atomen im Siloxan gebunden. Vorzugsweise steht n für Zahlen, die Polysiloxane beschreiben, welche Viskositäten zwischen 1 und 1.000.000 mm²/s besitzen, besonders bevorzugt Viskositäten zwischen 1.000 und 100.000 mm²/s.

Die Polysiloxane können zu einem geringen Grad verzweigt sein (beispielsweise < 2 Mol-% der Siloxaneinheiten), bzw sind die Polymere aber substantiell linear, besonders bevorzugt vollständig linear. Zudem können die Substituenten R ihrerseits substituiert sein, beispielsweise mit N-haltigen Gruppen (beispielsweise Aminogruppen), Epoxygruppen, S-haltige Gruppen, Si-haltige Gruppen, O-haltige Gruppen usw.. Vorzugsweise sind mindestens 80% der Reste R Alkylrste, besonders bevorzugt Methylgruppen.

Das Organosilikonmaterial, das mit dem Polysiloxan in der Kettenvedängerungsreaktion reagiert, kann entweder ein zweites Polysiloxan sein, oder ein Molekül, das als Kettenverlängerer agiert. Wenn das Organosilikonmaterial ein Polysiloxan ist, hat es die vorstehend erwähnte generelle Struktur. In diesen Fällen besitzt ein Polysiloxan in der Reaktion (mindestens) eine reaktive Gruppe, und ein zweites Polysiloxan besitzt (mindestens) eine zweite reaktive Gruppe, die mit der ersten reagiert.

Falls das Organosilikonmaterial ein Kettenverlängerungs-Agens umfaßt, kann dies ein materialsein wie beispielsweise ein Silan, ein Siloxan (beispielsweise Disiloxane oder Trisiloxan) oder ein Silazan. So kann beispielsweise eine Zusammensetzung, die ein Polysiloxan gemäß der vorstehend beschriebenen generellen Struktur umfaßt, welches mindestens eine Si-OH Gruppe aufweist, ketteverlängert werden, indem mit einem Alkoxysilan (beispielsweise einem Dialkoxysilan oder Trialkoxysilan) in Gegenwart von Zinn- oder Titan-haltigen Katalysatoren reagiert wird.

Die metallhaltigen Katalysatoren in der Kettenverlängerungsreaktion sind meist spezifisch für eine bestimmte Reaktion. Solche Katalysatoren sind im Stand der Technik bekannt und enthalten beispielsweise Metalle wie Platin, Rhodium, Zinn, Titan, Kupfer, Blei, etc.. In einer bevorzugten Kettenverlängerungsreaktion wird ein Polysiloxan mit mindestens einer aliphatisch ungesättigten Gruppe, vorzugsweise einer Endgruppe, mit einem Organosilikonmaterial in Gegenwart eines Hydrosilylierungskatalysators zur Reaktion gebracht, das ein Siloxan oder Polysiloxan mit mindestens einer (vorzugsweise endständigen) Si-H Gruppe ist. Das Polysiloxan besitzt mindestens eine aliphatisch ungesättigte Gruppe und genügt der allgemeinen oben angegeben Formel, in der R und n wie vortehend definiert sind, wobei im Durchschnitt zwischen 1 und 2 Gruppen R eine aliphatisch ungesättigte Gruppe pro Polymer besitzen. Repräsentative aliphatisch ungesättigte Gruppen sind beispielsweise Vinyl, Allyl, Hexenyl und Cyclohexenyl oder eine Gruppe R²CH=CHR³, in der R² für eine divalente aliphatische an das Silicium gebundene Kette und R³ für ein Wasserstoffatom oder eine Alkylgruppe steht. Das Organosilikonmaterial mit mindestens einer Si-H Gruppe hat vorzugsweise die oben genannte Struktur, in der R und n wie vorstehend definiert sind und wobei im Durchschnitt zwischen 1 und 2 Gruppen R ein Wasserstoff bedeuten und n 0 oder eine positive ganze Zahl ist

Dieses Material kann ein Polymer oder ein niedermolekulares Material wie ein Siloxan sein (beispielsweise ein Disiloxane oder ein Trisiloxan).

Das Polysiloxan mit mindestens einer aliphatisch ungesättigten Gruppe und das Organosilikonmaterial mit mindestens einer Si-H Gruppe reagieren in Gegenwart eines Hydrosilylierungskatalysators. Solche Katalysatoren sind aus dem Stand der Technik bekannt und umfassen beispielsweise Platin- und Rhodium-enthaltende Materialien. Die Katalysatoren können jede bekannte Form annehmen, beispielsweise auf Trägermaterialien (wie beispielsweise Silica Gel oder Aktivkohle) aufgebrachtes Platin oder Rhodium oder andere geeignete Compounds wie Platinchlorid, Salze von Platin-oder Chloroplatinsäuren. Ein wegen der guten Dispergierbarkeit in Organosilikonsystemen und der geringen Farbveränderungen bevorzugter Katalysator ist Chloroplatinsäure entweder als kommerziell verfügbares Hexahydrat oder in wasserfreier Form.

Bei einer weiteren bevorzugten Kettenerweiterungsreaktion wird ein Polysiloxan mit mindestens einer Si-OH Gruppe, vorzugsweise einer Endgruppe, mit einem Organosilikonmaterial zu Reaktion gebracht, das mindestens eine Alkoxygruppe besitzt, vorzugsweise ein Siloxan mit mindestens einer Si-OR Gruppe oder ein Alkoxysilan mit mindestens zwei Alkoxygruppen. Hierbei wird als Katalysator wieder ein metallhaltiger Katalysator eingesetzt.

Für die Reaktion einer Si-OH Gruppe mit einer Si-OR Gruppe existieren viele literaturbekannte Katalysatoren, beispielsweise Organometallverbindungen wie Organozinnsalze, Titanate oder Titanchelate bzw. -komplexe. Beispiele umfassen Zinnoctoat, Dibutylzinn-dilaurat, Dibutylzinn-diacetat, Dimethyltinn-dineodecanoat, Dibutylzinn-dimethoxid, Isobutylzinn-triceroat, Dimethylzinn-dibutyrat, Dimethylzinndineodecanoat, Triethylzinn-tartrat, Zinnoleat, Zinnnaphthenat, Zinnbutyrat, Zinnacetat, Zinnbenzoat, Zinnsebacat, Zinnsuccinat, Tetrabutyltitanat, Tetraisopropyltitante, Tetraphenyltitant, Tetraoctadecyltitanat, Titan-naphthanat, Ethyltriethanolamin-Titanat, Titani-diisopropyl-diethyl-acetoacetat, Titan-diisopropoxy-diacetyl-acetonat und Titanitetra-Alkoxide, bei denen das Alkoxid Butoxy oder Propoxy ist.

Die Silikon-in-Wasser-Emulsionen enthaltene darüber hinaus vorzugsweise mindestens ein Tensid. Diese wurden weiter oben ausführlich beschrieben.

Erfindungsgemäß ebenfalls bevorzugte Haarreinigungsmittel sind dadurch gekennzeichnet, daß sie mindestens ein Silikon der Formel IV

**R₃Si-[O-SiR₂]ₓ-(CH₂)ₙ-[O-SiR₂]_{y}-O-SiR₃** (IV),

enthalten, in der R für gleiche oder verschiedene Reste aus der Gruppe -H, -Phenyl, -Benzyl, -CH₂-CH(CH₃)Ph, der C₁₋₂₀-Alkylreste, vorzugsweise -CH₃, -CH₂CH₃, -CH₂CH₂CH₃, -CH(CH₃)₂, -CH₂CH₂CH₂H₃, -CH₂CH(CH₃)₂, -CH(CH3)CH₂CH₃, -C(CH₃)₃, steht, x bzw. y für eine Zahl von 0 bis 200, vorzugsweise von 0 bis 10, weiter bevorzugt von 0 bis 7 und insbesondere 0, 1, 2, 3, 4, 5 oder 6, stehen, und n für eine Zahl von 0 bis 10, bevorzugt von 1 bis 8 und insbesondere für 2, 3, 4, 5, 6 steht.

Mit Vorzug sind die Silikone wasserlöslich. Erfindungsgemäß bevorzugte Haarreinigungsmittel sind dadurch gekennzeichnet, daß sie mindestens ein wasserlösliches Silikon enthalten.

Eine weitere bevorzugte Gruppe von Inhaltsstoffen der erfindungsgemäßen Haarreinigungsmittel sind Vitamine, Provitamine oder Vitaminvorstufen. Diese werden nachfolgend beschrieben:

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäßen Mittel enthalten die Vitamin A-Komponente bevorzugt in Mengen von 0,05-1 Gew.-%, bezogen auf die gesamte Zubereitung.

Zur Vitamin B-Gruppe oder zu dem Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäß verwendetenen Mitteln bevorzugt in Mengen von 0,05 bis 1 Gew.-%, bezogen auf das gesamte Mittel, enthalten ist.
- Vitamin B₅ (Pantothensäure, Panthenol und Pantolacton). Im Rahmen dieser Gruppe wird bevorzugt das Panthenol und/oder Pantolacton eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 offenbarten kationischen Panthenolderivate. Die genannten Verbindungen des Vitamin B₅-Typs sind in den erfindungsgemäßen Mitteln bevorzugt in Mengen von 0,05 - 10 Gew.-%, bezogen auf das gesamte Mittel, enthalten. Mengen von 0,1 - 5 Gew.-% sind besonders bevorzugt.
- Vitamin B₆ (Pyridoxin sowie Pyridoxamin und Pyridoxal).
   Vitamin C (Ascorbinsäure). Vitamin C wird in den erfindungsgemäßen Mitteln bevorzugt in Mengen von 0,1 bis 3 Gew.-%, bezogen auf das gesamte Mittel eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäßen Mitteln bevorzugt in Mengen von 0,05-1 Gew.-%, bezogen auf das gesamte Mittel, enthalten. Vitamin F. Unter dem Begriff "Vitamin F" werden üblicherweise essentielle Fettsäuren, insbesondere Linolsäure, Linolensäure und Arachidonsäure, verstanden.

Vitamin H. Als Vitamin H wird die Verbindung (3aS, 4S, 6a*R*)-2-Oxohexahydrothienol[3,4-*d*]-imidazol-4-valeriansäure bezeichnet, für die sich aber inzwischen der Trivialname Biotin durchgesetzt hat. Biotin ist in den erfindungsgemäßen Mitteln bevorzugt in Mengen von 0,0001 bis 1,0 Gew.-%, insbesondere in Mengen von 0,001 bis 0,01 Gew.-% enthalten.

Zusammenfassend sind erfindungsgemäße Haarreinigungsmittel bevorzugt, die Vitamine, Pro-Vitamine und Vitaminvorstufen enthält, die den Gruppen A, B, C, E, F und H zugeordnet werden, wobei bevorzugte Zusammensetzungen die genannten Verbindungen in Mengen von von 0,1 bis 5 Gew.-%, vorzugsweise von 0,25 bis 4 Gew.-% und insbesondere von 0,5 bis 2,5 Gew.-%, jeweils bezogen auf das gesamte Mittel, enthalten.

In einer weiteren bevorzugten Ausführungsform können die erfindungsgemäßen Mittel Emulgatoren (F) enthalten. Emulgatoren bewirken an der Phasengrenzfläche die Ausbildung von wasser- bzw. ölstabilen Adsorptionsschichten, welche die dispergierten Tröpfchen gegen Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W - Emulsionen und hydrophobe Emulgatoren bilden bevorzugt W/O - Emulsionen. Unter einer Emulsion ist eine tröpfchenförmige Verteilung (Dispersion) einer Flüssigkeit in einer anderen Flüssigkeit unter Aufwand von Energie zur Schaffung von stabilisierenden Phasengrenzflächen mittels Tensiden zu verstehen. Die Auswahl dieser emulgierenden Tenside oder Emulgatoren richtet sich dabei nach den zu dispergierenden Stoffen und der jeweiligen äußeren Phase sowie der Feinteiligkeit der Emulsion. Erfindungsgemäß verwendbare Emulgatoren sind beispielsweise
- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov® 68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls® PGPH),
- Lineare und verzweigte Fettsäuren mit 8 bis 30 C -Atomen und deren Na-, K-, Ammonium-, Ca-, Mg- und Zn - Salze.

Die erfindungsgemäßen Mittel enthalten die Emulgatoren bevorzugt in Mengen von 0,1 - 25 Gew.-%, insbesondere 0,5 - 15 Gew.-%, bezogen auf das gesamte Mittel.

Bevorzugt können die erfindungsgemäßen Zusammensetzungen mindestens einen nichtionogenen Emulgator mit einem HLB-Wert von 8 bis 18 enthalten. Nichtionogene Emulgatoren mit einem HLB-Wert von 10-15 können erfindungsgemäß besonders bevorzugt sein.

Als weiterhin vorteilhaft hat es sich gezeigt, wenn Polymere (G) in den erfindungsgemäßen Mitteln enthalten sind. In einer bevorzugten Ausführungsform werden den erfindungsgemäß verwendeten Mitteln daher Polymere zugesetzt, wobei sich sowohl kationische, anionische, amphotere als auch nichtionische Polymere als wirksam erwiesen haben.

Erfindungsgemäß einsetzbar sind vorzugsweise kationische bzw. amphotere Polymere. Unter kationischen bzw. amphoteren Polymeren sind Polymere zu verstehen, welche in der Haupt- und/oder Seitenkette eine Gruppe aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C1-4-Kohlenwasserstoffgruppe an eine aus Acrylsäure, Methacrylsäure oder deren Derivaten aufgebaute Polymerhauptkette gebunden sind, haben sich als besonders geeignet erwiesen.

Homopolymere der allgemeinen Formel (G1-I), 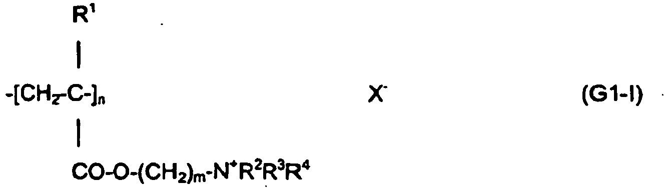 in der R¹= -H oder -CH₃ ist, R², R³ und R⁴ unabhängig voneinander ausgewählt sind aus C1-4-Alkyl-, -Alkenyl- oder -Hydroxyalkylgruppen, m = 1, 2, 3 oder 4, n eine natürliche Zahl und X⁻ ein physiologisch verträgliches organisches oder anorganisches Anion ist, sowie Copolymere, bestehend im wesentlichen aus den in Formel (G1-I) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:
- R¹ steht für eine Methylgruppe
- R², R³ und R⁴ stehen für Methylgruppen
- m hat den Wert 2.

Als physiologisch verträgliches Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCI-Bezeichnung Polyquatemium-37. Solche Produkte sind beispielsweise unter den Bezeichnungen Rheocare® CTH (Cosmetic Rheologies) und Synthalen® CR (Ethnichem) im Handel erhältlich. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare® SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare® SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylenether (INCI-Bezeichnung: PPG-1-Trideceth-6)) im Handel erhältlich.

Copolymere mit Monomereinheiten gemäß Formel (G1-I) enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare® SC 92 erhältlich.

Weitere bevorzugte kationische Polymere sind beispielsweise
- quaternisierte Cellulose-Derivate, wie sie unter den Bezeichnungen Celquat® und Polymer JR® im Handel erhältlich sind. Die Verbindungen Celquat® H 100, Celquat® L 200 und Polymer JR® 400 (INCI-Bezeichnung: Polyquaternium 10) sind bevorzugte quaternierte Cellulose-Derivate,
- kationische Alkylpolyglycoside gemäß der DE-PS 44 13 686,
- kationiserter Honig, beispielsweise das Handelsprodukt Honeyquat® 50,
- kationische Guar-Derivate, wie insbesondere die unter den Handelsnamen Cosmedia® Guar und Jaguar® vertriebenen Produkte,
- polymere Dimethyldiallylammoniumsalze und deren Copolymere mit Estern und Amiden von Acrylsäure und Methacrylsäure. Die unter den Bezeichnungen Merquat® 100 (Poly(dimethyldiallylammoniumchlorid)) und Merquat® 550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat® 734 und Gafquat® 755 im Handel erhältlich,
- Vinylpyrrolidon-Vinylimidazoliummethochlorid-Copolymere, wie sie unter den Bezeichnungen Luviquat® FC 370, FC 550, FC 905 und HM 552 angeboten werden,
- quaternierter Polyvinylalkohol,
- sowie die unter den Bezeichnungen Polyquaternium 2, Polyquaternium 17, Polyquaternium 18 und Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft® LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix® VC 713 (Hersteller: ISP), Gafquat® ASCP 1011, Gafquat® HS 110, Luviquat® 8155 und Luviquat® MS 370 erhältlich sind.

Weitere in den erfindungsgemäßen Mitteln einsetzbare kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen® CMF, Hydagen® HCMF, Kytamer® PC und Chitolam® NB/101 im Handel frei verfügbar sind.

Erfindungsgemäß bevorzugte kationische Polymere sind kationische Cellulose-Derivate und Chitosan und dessen Derivate, insbesondere die Handelsprodukte Polymer® JR 400, Hydagen® HCMF und Kytamer® PC, kationische Guar-Derivate, kationische Honig-Derivate, insbesondere das Handelsprodukt Honeyquat® 50, kationische Alkylpolyglycodside gemäß der DE-PS 44 13 686 und Polymere vom Typ Polyquaternium-37.

Weiterhin sind kationiserte Proteinhydrolysate zu den kationischen Polymeren zu zählen, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder biotechnologisch gewonnenen Proteinhydrolysaten, stammen kann. Die den erfindungsgemäßen kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder einer Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quarternären Ammoniumsalzen wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein. Als typische Beispiele für die erfindungsgemäßen kationischen Proteinhydrolysate und -derivate seien die unter den INCI - Bezeichnungen im "International Cosmetic Ingredient Dictionary and Handbook", (seventh edition 1997, The Cosmetic, Toiletry, and Fragrance Association 1101 17th Street, N.W., Suite 300, Washington, DC 20036-4702) genannten und im Handel erhältlichen Produkte genannt: Cocodimonium Hydroxypropyl Hydrolyzed Collagen, Cocodimopnium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Soy Protein, Cocodimonium Hydroxypropyl Hydrolyzed Wheat Protein, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed Keratin, Hydroxypropyltrimonium Hydrolyzed Rice Bran Protein, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Soy Protein, Quaternium-79 Hydrolyzed Wheat Protein.

Ganz besonders bevorzugt sind die kationischen Proteinhydrolysate und -derivate auf pflanzlicher Basis.

Erfindungsgemäß bevorzugte Haarreinigungsmittel sind dadurch gekennzeichnet, daß sie zusätzlich 0,05 bis 10 Gew.-%, vorzugsweise 0,1 bis 5 Gew.-%, besonders bevorzugt 0,2 bis 4 Gew.-% und insbesondere 0,3 bis 3 Gew.-% kationische(s) Polymer(e), vorzugsweise kationische(s) Cellulosederivat(e) und/oder kationische(s) Guar-Derivat(e), insbesondere Polyquatemium-10, enthalten.

Zusätzlich zu kationischen Polymerisaten oder an ihrer Stelle können die erfindungsgemäßen Mittel auch amphotere Polymere enthalten. Diese weisen zusätzlich mindestens eine negativ geladene Gruppe im Molekül auf und werden auch als zwitterionische Polymere bezeichnet. Im Rahmen der vorliegenden Erfindung bevorzugt einsetzbare zwitterionische Polymerisate setzen sich im wesentlichen zusammen aus
A) Monomeren mit quartären Ammoniumgruppen der allgemeinen Formel (Z-I),

   R¹-CH=CR²-CO-Z-(CₙH₂ₙ)-N⁽⁺⁾R³R⁴R⁵ A⁽⁻⁾ (Z-I)

   In der R¹ und R² unabhängig voneinander stehen für Wasserstoff oder eine Methylgruppe und R³, R⁴ und R⁵ unabhängig voneinander für Alkylgruppen mit 1 bis 4 Kohlenstoff-Atomen, Z eine NH-Gruppe oder ein Sauerstoffatom, n eine ganze Zahl von 2 bis 5 und A(-) das Anion einer organischen oder anorganischen Säure ist und
B) monomeren Carbonsäuren der allgemeinen Formel (Z-II),

   R₆-CH=CR⁷-COOH (II)

   in denen R⁶ und R⁷ unabhängig voneinander Wasserstoff oder Methylgruppen sind.

Geeignete Ausgangsmonomere sind z. B. Dimethylaminoethylacrylamid, Dimethylaminoethylmethacrylamid, Dimethylaminopropylacrylamid, Dimethylaminopropylmethacrylamid und Diethylaminoethylacrylamid, wenn Z eine NH-Gruppe bedeutet oder Dimethylaminoethylacrylat, Dimethylaminoethylmethacrylat und Diethylaminoethylacrylat, wenn Z ein Sauerstoffatom ist.

Die eine tertiäre Aminogruppe enthaltenden Monomeren werden dann in bekannter Weise quarterniert, wobei als Alkylierungsreagenzien Methylchlorid, Dimethylsulfat oder Diethylsulfat besonders geeignet sind. Die Quaternisierungsreaktion kann in wäßriger Lösung oder im Lösungsmittel erfolgen.

Vorteilhafterweise werden solche Monomere der Formel (Z-I) verwendet, die Derivate des Acrylamids oder Methacrylamids darstellen. Weiterhin bevorzugt sind solche Monomeren, die als Gegenionen Halogenid-, Methoxysulfat- oder Ethoxysulfat-tonen enthalten. Ebenfalls bevorzugt sind solche Monomeren der Formel (Z-I), bei denen R³, R⁴ und R⁵ Methylgruppen sind.

Das Acrylamidopropyl-trimethylammoniumchlorid ist ein ganz besonders bevorzugtes Monomer der Formel (Z-I).

Als monomere Carbonsäuren der Formel (Z-II) eignen sich Acrylsäure, Methacrylsäure, Crotonsäure und 2-Methyl-crotonsäure. Bevorzugt werden Acryl- oder Methacrylsäure, insbesondere Acrylsäure, eingesetzt.

Die erfindungsgemäß einsetzbaren zwitterionischen Polymerisate werden aus Monomeren der Formeln (Z-I) und (Z-II) nach an sich bekannten Polymerisationsverfahren hergestellt. Die Polymerisation kann entweder in wäßriger oder wäßrigalkoholischer Lösung erfolgen. Als Alkohole werden Alkohole mit 1 bis 4 Kohlenstoffatomen, vorzugsweise Isopropanol, verwendet, die gleichzeitig als Polymerisationsregler dienen. Der Monomerlösung können aber auch andere Komponenten als Regler zugesetzt werden, z. B. Ameisensäure oder Mercaptane, wie Thioethanol und Thioglykolsäure. Die Initiierung der Polymerisation erfolgt mit Hilfe von radikalbildenden Substanzen. Hierzu können Redoxsysteme und/oder thermisch zerfallende Radikalbildner vom Typ der Azoverbindungen, wie z. B. Azoisobuttersäurenitril, Azo-bis-(cyanopentansäure) oder Azo-bis-(amidinopropan)dihydrochlorid verwendet werden. Als Redoxsysteme eignen sich z. B. Kombinationen aus Wasserstoffperoxid, Kalium- oder Ammoniumperoxodisulfat sowie tertiäres Butylhydroperoxid mit Natriumsulfit, Natriumdithionit oder Hydroxylaminhydrochlorid als Reduktionskomponente.

Die Polymerisation kann isotherm oder unter adiabatischen Bedingungen durchgeführt werden, wobei in Abhängigkeit von den Konzentrationsverhältnissen durch die freiwerdende Polymerisationswärme der Temperaturbereich für den Ablauf der Reaktion zwischen 20 und 200 °C schwanken kann, und die Reaktion gegebenenfalls unter dem sich einstellenden Überdruck durchgeführt werden muß. Bevorzugterweise liegt die Reaktionstemperatur zwischen 20 und 100 °C.

Der pH-Wert während der Copolymerisation kann in einem weiten Bereich schwanken. Vorteilhafterweise wird bei niedrigen pH-Werten polymerisiert; möglich sind jedoch auch pH-Werte oberhalb des Neutralpunktes. Nach der Polymerisation wird mit einer wäßrigen Base, z. B. Natronlauge, Kalilauge oder Ammoniak, auf einen pH-Wert zwischen 5 und 10, vorzugsweise 6 bis 8, eingestellt. Nähere Angaben zum Polymerisationsverfahren können den Beispielen entnommen werden.

Als besonders wirksam haben sich solche Polymerisate erwiesen, bei denen die Monomeren der Formel (Z-I) gegenüber den Monomeren der Formel (Z-II) im Überschuß vorlagen. Es ist daher erfindungsgemäß bevorzugt, solche Polymerisate zu verwenden, die aus Monomeren der Formel (Z-I) und die Monomeren der Formel (Z-II) in einem Molverhältnis von 60:40 bis 95:5, insbesondere von 75:25 bis 95:5, bestehen.

Die kationischen bzw. amphoteren Polymere sind in den erfindungsgemäßen Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Bei den anionischen Polymeren (G2) handelt es sich um anionische Polymere, welche Carboxylat- und/oder Sulfonatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure.

Als ganz besonders wirkungsvoll haben sich anionische Polymere erwiesen, die als alleiniges oder Co-Monomer 2-Acrylamido-2-methylpropansulfonsäure enthalten, wobei die Sulfonsäuregruppe ganz oder teilweise als Natrium-, Kalium-, Ammonium-, Mono- oder Triethanolammonium-Salz vorliegen kann.

Besonders bevorzugt ist das Homopolymer der 2-Acrylamido-2-methylpropansulfonsäure, das beispielsweise unter der Bezeichnung Rheothik® 11-80 im Handel erhältlich ist.

Innerhalb dieser Ausführungsform kann es bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester.
Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylen-bisacrylamid zum Einsatz kommen. Ein solches Polymer ist in dem Handelsprodukt Sepigel® 305 der Firma SEPPIC enthalten. Die Verwendung dieses Compounds, das neben der Polymerkomponente eine Kohlenwasserstoffmischung (C₁₃-C₁₄-Isoparaffin) und einen nichtionogenen Emulgator (Laureth-7) enthält, hat sich im Rahmen der erfindungsgemäßen Lehre als besonders vorteilhaft erwiesen.

Auch die unter der Bezeichnung Simulgel® 600 als Compound mit Isohexadecan und Polysorbat-80 vertriebenen Natriumacryloyldimethyltaurat-Copolymere haben sich als erfindungsgemäß besonders wirksam erwiesen.

Ebenfalls bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichen Carbopol® im Handel erhältlich.

Copolymere aus Maleinsäureanhydrid und Methylvinylether, insbesondere solche mit Vernetzungen, sind ebenfalls farberhaltende Polymere. Ein mit 1,9-Decadiene vernetztes Maleinsäure-Methylvinylether-Copolymer ist unter der Bezeichnung Stabileze® QM im Handel erhältlich.

Weiterhin können als Polymere zur Steigerung der Wirkung des erfindungsgemäßen Wirkstoffkomplexes (A) amphotere Polymere (G3) verwendet werden. Unter dem Begriff amphotere Polymere werden sowohl solche Polymere, die im Molekül sowohl freie Aminogruppen als auch freie -COOH- oder SO₃H-Gruppen enthalten und zur Ausbildung innerer Salze befähigt sind, als auch zwitterionische Polymere, die im Molekül quartäre Ammoniumgruppen und -COO⁻- oder -SO₃⁻-Gruppen enthalten, und solche Polymere zusammengefaßt, die -COOH- oder SO₃H-Gruppen und quartäre Ammoniumgruppen enthalten.

Ein Beispiel für ein erfindungsgemäß einsetzbares Amphopolymer ist das unter der Bezeichnung Amphomer® erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt.

Bevorzugt eingesetzte amphotere Polymere sind solche Polymerisate, die sich im wesentlichen zusammensetzen aus
(a) Monomeren mit quartären Ammoniumgruppen der allgemeinen Formel (G3-I),

   R¹-CH=CR²-CO-Z-(CₙH₂ₙ)-N⁽⁺⁾R³R⁴R⁵ A⁽⁻⁾ (G3-I)

   in der R¹ und R² unabhängig voneinander stehen für Wasserstoff oder eine Methylgruppe und R³, R⁴ und R⁵ unabhängig voneinander für Alkylgruppen mit 1 bis 4 Kohlenstoffatomen, Z eine NH-Gruppe oder ein Sauerstoffatom, n eine ganze Zahl von 2 bis 5 und A⁽⁻⁾ das Anion einer organischen oder anorganischen Säure ist, und
(b) monomeren Carbonsäuren der allgemeinen Formel (G3-II),

   R⁶-CH=CR⁷-COOH (G3-II)

   in denen R⁶ und R⁷ unabhängig voneinander Wasserstoff oder Methylgruppen sind.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Ganz besonders bevorzugt sind solche Polymerisate, bei denen Monomere des Typs (a) eingesetzt werden, bei denen R³, R⁴ und R⁵ Methylgruppen sind, Z eine NH-Gruppe und A⁽⁻⁾ ein Halogenid-, Methoxysulfat- oder Ethoxysulfat-Ion ist; Acrylamidopropyl-trimethyl-ammoniumchlorid ist ein besonders bevorzugtes Monomeres (a). Als Monomeres (b) für die genannten Polymerisate wird bevorzugt Acrylsäure verwendet.

Die erfindungsgemäßen Mittel können in einer weiteren Ausführungsform nichtionogene Polymere (G4) enthalten.

Geeignete nichtionogene Polymere sind beispielsweise:
- Vinylpyrrolidon/Vinylester-Copolymere, wie sie beispielsweise unter dem Warenzeichen Luviskol® (BASF) vertrieben werden. Luviskol® VA 64 und Luviskol® VA 73,
- jeweils Vinylpyrrolidon/Vinylacetat-Copolymere, sind ebenfalls bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminal® und Benecel® (AQUALON) und Natrosol®-Typen (Hercules) vertrieben werden.
- Stärke und deren Derivate, insbesondere Stärkeether, beispielsweise Structure® XL (National Starch), eine multifunktionelle, salztolerante Stärke;
- Schellack
- Polyvinylpyrrolldone, wie sie beispielsweise unter der Bezeichnung Luviskol® (BASF) vertrieben werden.
- Siloxane. Diese Siloxane können sowohl wasserlöslich als auch wasserunlöslich sein. Geeignet sind sowohl flüchtige als auch nichtflüchtige Siloxane, wobei als nichtflüchtige Siloxane solche Verbindungen verstanden werden, deren Siedepunkt bei Normaldruck oberhalb von 200 °C liegt. Bevorzugte Siloxane sind Polydialkylsiloxane, wie beispielsweise Polydimethylsiloxan, Polyalkylarylsiloxane, wie beispielsweise Polyphenylmethylsiloxan, ethoxylierte Polydialkylsiloxane sowie Polydialkylsiloxane, die Amin- und/oder Hydroxy-Gruppen enthalten.
- Glycosidisch substituierte Silicone.

Es ist erfindungsgemäß auch möglich, daß die verwendeten Zubereitungen mehrere, insbesondere zwei verschiedene Polymere gleicher Ladung und/oder jeweils ein ionisches und ein amphoteres und/oder nicht ionisches Polymer enthalten.

Die Polymere (G) sind in den erfindungsgemäßen Mitteln bevorzugt in Mengen von 0,05 bis 10 Gew.-%, bezogen auf das gesamte Mittel, enthalten. Mengen von 0,1 bis 5, insbesondere von 0,1 bis 3 Gew.-%, sind besonders bevorzugt.

Zusätzlich zu den genannten Stoffen können die erfindungsgemäßen Mittel weitere Pflegestoffe enthalten.

Eine weitere Gruppe von Pflegestoffen, die in den erfindungsgemäßen Mitteln enthalten sein kann, sind die Proteinhydrolysate und deren Derivate (P) enthalten. Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Weiterhin werden erfindungsgemäß aus Aminosäuren und Aminosäurederivaten aufgebaute Polymere unter dem Begriff Proteinhydrolysate verstanden. Zu letzteren sind beispielsweise Polyalanin, Polyasparagin, Polyserin etc. zu zählen. Weitere Beispiele für erfindungsgemäß einsetzbare Verbindungen sind L-Alanyl-L-prolin, Polyglycin, Glycyl-L-glutamin oder D/L-Methionin-S-Methylsulfoniumchlorid. Selbstverständlich können erfindungsgemäß auch β-Aminosäuren und deren Derivate wie β-Alanin, Anthranilsäure oder Hippursäure eingesetzt werden. Das Molgewicht der erfindungsgemäß einsetzbaren Proteinhydrolysate liegt zwischen 75, dem Molgewicht für Glycin, und 200000, bevorzugt beträgt das Molgewicht 75 bis 50000 und ganz besonders bevorzugt 75 bis 20000 Dalton.

Erfindungsgemäß können Proteinhydrolysate sowohl pflanzlichen als auch tierischen oder marinen oder synthetischen Ursprungs eingesetzt werden.

Tierische Proteinhydrolysate sind beispielsweise Elastin-, Kollagen-, Keratin- und Milcheiweiß-Proteinhydrolysate, die auch in Form von Salzen vorliegen können. Solche Produkte werden beispielsweise unter den Warenzeichen Dehylan® (Cognis), Promois® (Interorgana), Collapuron® (Cognis), Nutrilane® (Cognis), Gelita-Sol® (Deutsche Gelatine Fabriken Stoess & Co), Lexein® (Inolex) und Kerasol® (Croda) vertrieben.

Erfindungsgemäß bevorzugt ist die Verwendung von Proteinhydrolysaten pflanzlichen Ursprungs, z. B. Soja-, Mandel-, Erbsen-, Kartoffel- und Weizenproteinhydrolysate. Solche Produkte sind beispielsweise unter den Warenzeichen Gluadin® (Cognis), DiaMin® (Diamalt), Lexein® (Inolex), Hydrosoy® (Croda), Hydrolupin® (Croda), Hydrosesame® (Croda), Hydrotritium® (Croda) und Crotein® (Croda) erhältlich.

Wenngleich der Einsatz der Proteinhydrolysate als solche bevorzugt ist, können an deren Stelle gegebenenfalls auch anderweitig erhaltene Aminosäuregemische eingesetzt werden. Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon® (Cognis), Lexein® (Inolex), Crolastin® (Croda) oder Crotein® (Croda) vertrieben.

Selbstverständlich umfaßt die erfindungsgemäße Lehre alle isomeren Formen, wie cis - trans - Isomere, Diastereomere und chirale Isomere.

Erfindungsgemäß ist es auch möglich, eine Mischung aus mehreren Proteinhydrolysaten (P) einzusetzen.

Die Proteinhydrolysate (P) sind in den Mitteln in Konzentrationen von 0,01 Gew.% bis zu 20 Gew.%, vorzugsweise von 0,05 Gew.% bis zu 15 Gew.% und ganz besonders bevorzugt in Mengen von 0,05 Gew.% bis zu 5 Gew.% enthalten.

Weiterhin kann in einer bevorzugten Ausführungsform der Erfindung ein erfindungsgemäßes Mittel auch UV - Filter (I) enthalten. Die erfindungsgemäß zu verwendenden UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.
Die erfindungsgemäß verwendeten UV-Filter können beispielsweise ausgewählt werden aus substituierten Benzophenonen, p-Aminobenzoesäureestem, Diphenylacrylsäureestern, Zimtsäureestern, Salicylsäureestern, Benzimidazolen und o-Aminobenzoesäureestern.

Beispiele für erfindungsgemäß verwendbar UV-Filter sind 4-Amino-benzoesäure, N,N,N-Trimethyl-4-(2-oxoborn-3-ylidenmethyl)anilin-methylsulfat, 3,3,5-Trimethyl-cyclohexylsalicylat (Homosalate), 2-Hydroxy-4-methoxy-benzophenon (Benzophenone-3; Uvinul® M 40, Uvasorb® MET, Neo Heliopan® BB, Eusolex® 4360), 2-Phenylbenzimidazol-5-sulfonsäure und deren Kalium-, Natrium- und Triethanolaminsalze (Phenylbenzimidazole sulfonic acid; Parsol® HS; Neo Heliopan® Hydro), 3,3'-(1,4-Phenylendimethylen)-bis(7,7-dimethyl-2-oxo-bicyclo-[2.2.1]hept-1-yl-methan-sulfonsäure) und deren Salze, 1-(4-tert.-Butylphenyl)-3-(4-methoxyphenyl)-propan-1,3-dion (Butyl methoxydibenzoylmethane; Parsol® 1789, Eusolex® 9020), α-(2-Oxoborn-3-yliden)-toluol-4-sulfonsäure und deren Salze, ethoxylierte 4-Aminobenzoesäure-ethylester (PEG-25 PABA; Uvinul® P 25), 4-Dimethylaminobenzoesäure-2-ethylhexylester (Octyl Dimethyl PABA; Uvasorb® DMO, Escalol® 507, Eusotex® 6007), Salicylsäure-2-ethylhexylester (Octyl Salicylat; Escalol® 587, Neo Heliopan® OS, Uvinul® 018), 4-Methoxyzimtsäure-isopentylester (Isoamyl p-Methoxycinnamate; Neo Heliopan® E 1000), 4-Methoxyzimtsäure-2-ethylhexyl-ester (Octyl Methoxycinnamate; Parsol® MCX, Escalol® 557, Neo Heliopan® AV), 2-Hydroxy-4-methoxybenzophenon-5-sulfonsäure und deren Natriumsalz (Benzophenone-4; Uvinul® MS 40; Uvasorb® S 5), 3-(4'-Methylbenzyliden)-D,L-Campher (4-Methylbenzylidene camphor; Parsol® 5000, Eusolex® 6300), 3-Benzyliden-campher (3-Benzylidene camphor), 4-Isopropylbenzylsalicylat, 2,4,6-Trianilino-(p-carbo-2'-ethylhexyl-1'-oxi)-1,3,5-triazin, 3-Imidazol-4-yl-acrylsäure und deren Ethylester, Polymere des N-{(2 und 4)-[2-oxoborn-3-ylidenmethyl]benzyl}-acrylamids, 2,4-Dihydroxybenzophenon (Benzophenone-1; Uvasorb® 20 H, Uvinul® 400), 1,1'-Diphenylacrylonitrilsäure-2-ethylhexyl-ester (Octocrylene; Eusolex® OCR, Neo Hetiopan® Type 303, Uvinul® N 539 SG), o-Aminobenzoesäure-menthylester (Menthyl Anthranilate; Neo Heliopan® MA), 2,2',4,4'-Tetrahydroxybenzophenon (Benzophenone-2; Uvinul® D-50), 2,2'-Dihydroxy-4,4'-dimethoxybenzophenon (Benzophenone-6), 2,2'-Dihydroxy-4,4'-dimethoxybenzophenon-5-natriumsulfonat und 2-Cyano-3,3-diphenylacrylsäure-2'-ethylhexylester. Bevorzugt sind 4-Aminobenzoesäure, N,N,N-Trimethyl-4-(2-oxobom-3-ylidenmethyl)anilin-methylsulfat, 3,3,5-Trimethyl-cyclohexylsalicylat, 2-Hydroxy-4-methoxy-benzophenon, 2-Phenylbenzimidazol-5-sulfonsäure und deren Kalium-, Natrium- und Triethanolaminsalze, 3,3'-(1,4-Phenylendimethylen)-bis(7,7-dimethyl-2-oxo-bicyclo-[2.2.1]hept-1-yl-methan-sulfonsäure) und deren Salze, 1-(4-tert.-Butylphenyl)-3-(4-methoxyphenyl)-propan-1,3-dion, α-(2-Oxoborn-3-yliden)-toluol-4-sulfonsäure und deren Salze, ethoxylierte 4-Aminobenzoesäure-ethylester, 4-Dimethylaminobenzoesäure-2-ethylhexylester, Salicylsäure-2-ethylhexylester, 4-Methoxyzimtsäure-isopentylester, 4-Methoxyzimtsäure-2-ethylhexylester, 2-Hydroxy-4-methoxybenzophenon-5-sulfonsäure und deren Natriumsalz, 3-(4'-Methylbenzyliden)-D,L-Campher, 3-Benzyliden-campher, 4-Isopropylbenzylsalicylat, 2,4,6-Trianilino-(p-carbo-2'-ethylhexyl-1'-oxi)-1,3,5-triazin, 3-Imidazol-4-yl-acrylsäure und deren Ethylester, Polymere des N-{(2 und 4)-[2-oxoborn-3-ylidenmethyl]benzyl}-acrylamid. Erfindungsgemäß ganz besonders bevorzugt sind 2-Hydroxy-4-methoxy-benzophenon, 2-Phenylbenzimidazol-5-sulfonsäure und deren Kalium-, Natrium- und Triethanolaminsalze, 1-(4-tert.-Butylphenyl)-3-(4-methoxyphenyl)-propan-1,3-dion, 4-Methoxyzimtsäure-2-ethylhexyl-ester und 3-(4'-Methylbenzyliden)-D,L-Campher.

Bevorzugt sind solche UV-Filter, deren molarer Extinktionskoeffizient am Absorptionsmaximum oberhalb von 15 000, insbesondere oberhalb von 20000, liegt.

Weiterhin wurde gefunden, daß bei strukturell ähnlichen UV-Filtern in vielen Fällen die wasserunlösliche Verbindung im Rahmen der erfindungsgemäßen Lehre die höhere Wirkung gegenüber solchen wasserlöslichen Verbindungen aufweist, die sich von ihr durch eine oder mehrere zusätzlich ionische Gruppen unterscheiden. Als wasserunlöslich sind im Rahmen der Erfindung solche UV-Filter zu verstehen, die sich bei 20 °C zu nicht mehr als 1 Gew.-%, insbesondere zu nicht mehr als 0,1 Gew.-%, in Wasser lösen. Weiterhin sollten diese Verbindungen in üblichen kosmetischen Ölkomponenten bei Raumtemperatur zu mindestens 0,1, insbesondere zu mindestens 1 Gew.% löslich sein). Die Verwendung wasserunlöslicher UV-Filter kann daher erfindungsgemäß bevorzugt sein.

Gemäß einer weiteren Ausführungsform der Erfindung sind solche UV-Filter bevorzugt, die eine kationische Gruppe, insbesondere eine quartäre Ammoniumgruppe, aufweisen.

Diese UV-Filter weisen die allgemeine Struktur U - Q auf.

Der Strukturteil U steht dabei für eine UV-Strahlen absorbierende Gruppe. Diese Gruppe kann sich im Prinzip von den bekannten, im Kosmetikbereich einsetzbaren, oben genannten UV-Filtern ableiten, in dem eine Gruppe, in der Regel ein Wasserstoffatom, des UV-Filters durch eine kationische Gruppe Q, insbesondere mit einer quartären Aminofunktion, ersetzt wird.
Verbindungen, von denen sich der Strukturteil U ableiten kann, sind beispielsweise
- substituierte Benzophenone,
- p-Aminobenzoesäureester,
- Diphenylacrylsäureester,
- Zimtsäureester,
- Salicylsäureester,
- Benzimidazole und
- o-Aminobenzoesäureester.

Strukturteile U, die sich vom Zimtsäureamid oder vom N,N-Dimethylamino-benzoesäureamid ableiten, sind erfindungsgemäß bevorzugt.

Die Strukturteile U können prinzipiell so gewählt werden, daß das Absorptionsmaximum der UV-Filter sowohl im UVA(315-400 nm)-, als auch im UVB(280-315nm)- oder im UVC(<280 nm)-Bereich liegen kann. UV-Filter mit einem Absorptionsmaximum im UVB-Bereich, insbesondere im Bereich von etwa 280 bis etwa 300 nm, sind besonders bevorzugt.

Weiterhin wird der Strukturteil U, auch in Abhängigkeit von Strukturteil Q, bevorzugt so gewählt, daß der molare Extinktionskoeffizient des UV-Filters am Absorptionsmaximum oberhalb von 15 000, insbesondere oberhalb von 20000, liegt.

Der Strukturteil Q enthält als kationische Gruppe bevorzugt eine quartäre Ammoniumgruppe. Diese quartäre Ammoniumgruppe kann prinzipiell direkt mit dem Strukturteil U verbunden sein, so daß der Strukturteil U einen der vier Substituenten des positiv geladenen Stickstoffatomes darstellt. Bevorzugt ist jedoch einer der vier Substituenten am positiv geladenen Stickstoffatom eine Gruppe, insbesondere eine Alkylengruppe mit 2 bis 6 Kohlenstoffatomen, die als Verbindung zwischen dem Strukturteil U und dem positiv geladenen Stickstoffatom fungiert.

Vorteilhafterweise hat die Gruppe Q die allgemeine Struktur -(CH₂)ₓ-N⁺R¹R²R³X⁻, in der x steht für eine ganze Zahl von 1 bis 4, R¹ und R² unabhängig voneinander stehen für C₁₋₄-Alkylgruppen, R³ steht für eine C₁₋₂₂-Alkylgruppe oder eine Benzylgruppe und X⁻ für ein physiologisch verträgliches Anion. Im Rahmen dieser allgemeinen Struktur steht x bevorzugt für die die Zahl 3, R¹ und R² jeweils für eine Methylgruppe und R³ entweder für eine Methylgruppe oder eine gesättigte oder ungesättigte, lineare oder verzweigte Kohlenwasserstoffkette mit 8 bis 22, insbesondere 10 bis 18, Kohlenstoffatomen.

Physiologisch verträgliche Anionen sind beispielsweise anorganische Anionen wie Halogenide, insbesondere Chlorid, Bromid und Fluorid, Sulfationen und Phosphationen sowie organische Anionen wie Lactat, Citrat, Acetat, Tartrat, Methosulfat und Tosylat.

Zwei bevorzugte UV-Filter mit kationischen Gruppen sind die als Handelsprodukte erhältlichen Verbindungen Zimtsäureamidopropyl-trimethylammoniumchlorid (Incroquat® UV-283) und Dodecyl-dimethylaminobenzamidopropyl-dimethylammoniumtosylat (Escalol® HP 610).

Selbstverständlich umfaßt die erfindungsgemäße Lehre auch die Verwendung einer Kombination von mehreren UV-Filtern. Im Rahmen dieser Ausführungsform ist die Kombination mindestens eines wasserunlöslichen UV-Filters mit mindestens einem UV-Filter mit einer kationischen Gruppe bevorzugt.

Die UV-Filter (I) sind in den erfindungsgemäß verwendeten Mitteln üblicherweise in Mengen 0,1-5 Gew.%, bezogen auf das gesamte Mittel, enthalten. Mengen von 0,4-2,5 Gew.% sind bevorzugt.

Die erfindungsgemäßen Mittel können weiterhin eine 2-Pyrrolidinon-5-carbonsäure und deren Derivate (J) enthalten. Bevorzugt sind die Natrium-, Kalium-, Calcium-, Magnesium- oder Ammoniumsalze, bei denen das Ammoniumion neben Wasserstoff eine bis drei C₁- bis C₄-Alkylgruppen trägt. Das Natriumsalz ist ganz besonders bevorzugt. Die eingesetzten Mengen in den erfindungsgemäßen Mitteln betragen vorzugsweise 0,05 bis 10 Gew.%, bezogen auf das gesamte Mittel, besonders bevorzugt 0,1 bis 5, und insbesondere 0,1 bis 3 Gew.%.

Schließlich können die erfindungsgemäßen Mittel auch Pflanzenextrakte (L) enthalten.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättem der Pflanze herzustellen.

Hinsichtlich der erfindungsgemäß verwendbaren Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Erfindungsgemäß sind vor allem die Extrakte aus Grünem Tee, Eichenrinde, Brennessel, Hamamelis, Hopfen, Henna, Kamille, Klettenwurzel, Schachtelhalm, Weißdorn, Lindenblüten, Mandel, Aloe Vera, Fichtennadel, Roßkastanie, Sandelholz, Wachotder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Besonders bevorzugt sind die Extrakte aus Grünem Tee, Eichenrinde, Brennessel, Hamamelis, Hopfen, Kamille, Klettenwurzel, Schachtelhalm, Lindenblüten, Mandel, Aloe Vera, Kokosnuß, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, Ginseng und Ingwerwurzel.

Ganz besonders für die erfindungsgemäße Verwendung geeignet sind die Extrakte aus Grünem Tee, Mandel, Aloe Vera, Kokosnuß, Mango, Aprikose, Limone, Weizen, Kiwi und Melone.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Weiterhin kann es bevorzugt sein, in den erfindungsgemäßen Mitteln Mischungen aus mehreren, insbesondere aus zwei, verschiedenen Pflanzenextrakten einzusetzen.

Zusätzlich kann es sich als vorteilhaft erweisen, wenn in den erfindungsgemäßen Mitteln Penetrationshilfsstoffe und/ oder Quellmittel (M) enthalten sind. Hierzu sind beispielsweise zu zählen Harnstoff und Harnstoffderivate, Guanidin und dessen Derivate, Arginin und dessen Derivate, Wasserglas, Imidazol und Dessen Derivate, Histidin und dessen Derivate, Benzylalkohol, Glycerin, Glykol und Glykolether, Propylenglykol und Propylenglykolether, beispielsweise Propylenglykolmonoethylether, Carbonate, Hydrogencarbonate, Diole und Triole, und insbesondere 1,2-Diole und 1,3-Diole wie beispielsweise 1,2-Propandiol, 1,2-Pentandiol, 1,2-Hexandiol, 1,2-Dodecandiol, 1,3-Propandiol, 1,6-Hexandiol, 1,5-Pentandiol, 1,4-Butandiol.

Vorteilhaft im Sinne der Erfindung können zusätzlich kurzkettige Carbonsäuren (N) in den erfindungsgemäßen Mitteln enthalten sein. Unter kurzkettigen Carbonsäuren und deren Derivaten im Sinne der Erfindung werden Carbonsäuren verstanden, welche gesättigt oder ungesättigt und/oder geradkettig oder verzweigt oder cyclisch und/oder aromatisch und/oder heterocyclisch sein können und ein Molekulargewicht kleiner 750 aufweisen. Bevorzugt im Sinne der Erfindung können gesättigte oder ungesättigte geradkettigte oder verzweigte Carbonsäuren mit einer Kettenlänge von 1 bis zu 16 C-Atomen in der Kette sein, ganz besonders bevorzugt sind solche mit einer Kettenlänge von 1 bis zu 12 C - Atomen in der Kette.

Die kurzkettigen Carbonsäuren im Sinne der Erfindung können ein, zwei, drei oder mehr Carboxygruppen aufweisen. Bevorzugt im Sinne der Erfindung sind Carbonsäuren mit mehreren Carboxygruppen, insbesondere Di- und Tricarbonsäuren. Die Carboxygruppen können ganz oder teilweise als Ester, Säureanhydrid, Lacton, Amid, Imidsäure, Lactam, Lactim, Dicarboximid, Carbohydrazid, Hydrazon, Hydroxam, Hydroxim, Amidin, Amidoxim, Nitril, Phosphon- oder Phosphatester vorliegen. Die in den erfindungsgemäßen Mitteln enthaltenen Carbonsäuren können selbstverständlich entlang der Kohlenstoffkette oder des Ringgerüstes substituiert sein. Zu den Substituenten der erfindungsgemäßen Carbonsäuren sind beispielsweise zu zählen C1-C8-Alkyl-, C2-C8-Alkenyl-, Aryl-, Aralkyl- und Aralkenyl-, Hydroxymethyl-, C2-C8-Hydroxyalkyl-,C2-C8-Hydroxyalkenyl-, Aminomethyl-, C2-C8-Aminoalkyl-, Cyano-, Formyl-, Oxo-, Thioxo-, Hydroxy-, Mercapto-, Amino-, Carboxy- oder Iminogruppen. Bevorzugte Substituenten sind C1-C8-Alkyl-, Hydroxymethyl-, Hydroxy-, Amino- und Carboxygruppen. Besonders bevorzugt sind Substituenten in ⊏ - Stellung. Ganz besonders bevorzugte Substituenten sind Hydroxy-, Alkoxy- und Aminogruppen, wobei die Aminofunktion gegebenenfalls durch Alkyl-, Aryl-, Aralkyl- und/oder Alkenylreste weiter substituiert sein kann. Weiterhin sind ebenfalls bevorzugte Carbonsäurederivate die Phosphon- und Phosphatester.

Als Beispiele für in den erfindungsgemäßen Mitteln enthaltene Carbonsäuren seien genannt Ameisensäure, Essigsäure, Propionsäure, Buttersäure, Isobuttersäure, Valeriansäure, Isovaleriansäure, Pivalinsäure, Oxalsäure, Malonsäure, Bernsteinsäure, Glutarsäure, Glycerinsäure, Glyoxylsäure, Adipinsäure, Pimelinsäure, Korksäure, Azelainsäure, Sebacinsäure, Propiolsäure, Crotonsäure, Isocrotonsäure, Elaidinsäure, Maleinsäure, Fumarsäure, Muconsäure, Citraconsäure, Mesaconsäure, Camphersäure, Benzoesäure, o,m,p-Phthalsäure, Naphthoesäure, Toluoylsäure, Hydratropasäure, Atropasäure, Zimtsäure, Isonicotinsäure, Nicotinsäure, Bicarbaminsäure, 4,4'-Dicyano-6,6'-binicotinsäure, 8-Carbamoyloctansäure, 1,2,4-Pentantricarbonsäure, 2-Pyrrolcarbonsäure, 1,2,4,6,7-Napthalinpentaessigsäure, Malonaldehydsäure, 4-Hydroxyphthalamidsäure, 1-Pyrazolcarbonsäure, Gallussäure oder Propantricarbonsäure, eine Dicarbonsäure ausgewählt aus der Gruppe, die gebildet wird durch Verbindungen der allgemeinen Formel (N-1), 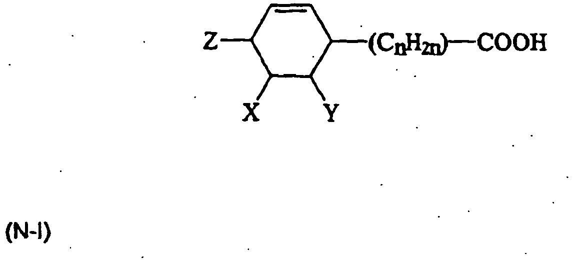 in der Z steht für eine lineare oder verzweigte Alkyl- oder Alkenylgruppe mit 4 bis 12 Kohlenstoffatomen, n für eine Zahl von 4 bis 12 sowie eine der beiden Gruppen X und Y für eine COOH-Gruppe und die andere für Wasserstoff oder einen Methyl- oder Ethylrest, Dicarbonsäuren der allgemeinen Formel (N-I), die zusätzlich noch 1 bis 3 Methyl- oder Ethylsubstituenten am Cyclohexenring tragen sowie Dicarbonsäuren, die aus den Dicarbonsäuren gemäß Formel (N-I) formal durch Anlagerung eines Moleküls Wasser an die Doppelbindung im Cyclohexenring entstehen.

Dicarbonsäuren der Formel (N-I) sind in der Literatur bekannt.

Die Dicarbonsäuren der Formel (N-I) können beispielsweise durch Umsetzung von mehrfach ungesättigten Dicarbonsäuren mit ungesättigten Monocarbonsäuren in Form einer Diels-Alder-Cyclisierung hergestellt werden. Üblicherweise wird man von einer mehrfach ungesättigten Fettsäure als Dicarbonsäurekomponente ausgehen. Bevorzugt ist die aus natürlichen Fetten und Ölen zugängliche Linolsäure. Als Monocarbonsäurekomponente sind insbesondere Acrylsäure, aber auch z.B. Methacrylsäure und Crotonsäure bevorzugt. Üblicherweise entstehen bei Reaktionen nach Diels-Alder Isomerengemische, bei denen eine Komponente im Überschuß vorliegt. Diese Isomerengemische können erfindungsgemäß ebenso wie die reinen Verbindungen eingesetzt werden.

Erfindungsgemäß einsetzbar neben den bevorzugten Dicarbonsäuren gemäß Formel (N-I) sind auch solche Dicarbonsäuren, die sich von den Verbindungen gemäß Formel (N-I) durch 1 bis 3 Methyl- oder Ethyl-Substituenten am Cyclohexylring unterscheiden oder aus diesen Verbindungen formal durch Anlagerung von einem Molekül Wasser an die Doppelbildung des Cyclohexenrings gebildet werden.

Als erfindungsgemäß besonders wirksam hat sich die Dicarbonsäure(-mischung) erwiesen, die durch Umsetzung von Linolsäure mit Acrylsäure entsteht. Es handelt sich dabei um eine Mischung aus 5- und 6-Carboxy-4-hexyl-2-cyclohexen-1-octansäure. Solche Verbindungen sind kommerziell unter den Bezeichnungen Westvaco Diacid® 1550 und Westvaco Diacid® 1595 (Hersteller: Westvaco) erhältlich.

Neben den zuvor beispielhaft aufgeführten erfindungsgemäßen kurzkettigen Carbonsäuren selbst können auch deren physiologisch verträgliche Salze erfindungsgemäß eingesetzt werden. Beispiele für solche Salze sind die Alkali-, Erdalkali-, Zinksalze sowie Ammoniumsalze, worunter im Rahmen der vorliegenden Anmeldung auch die Mono-, Di- und Trimethyl-, -ethyl- und -hydroxyethyl-Ammoniumsalze zu verstehen sind. Ganz besonders bevorzugt können im Rahmen der Erfindung jedoch mit alkalisch reagierenden Aminosäuren, wie beispielsweise Arginin, Lysin, Ornithin und Histidin, neutralisierte Säuren eingesetzt werden. Weiterhin kann es aus Formulierungsgründen bevorzugt sein, die Carbonsäure aus den wasserlöslichen Vertretern, insbesondere den wasserlöslichen Salzen, auszuwählen.

Weiterhin ist es erfindungsgemäß bevorzugt, Hydroxycarbonsäuren und hierbei wiederum insbesondere die Dihydroxy-, Trihydroxy- und Polyhydroxycarbonsäuren sowie die Dihydroxy-, Trihydroxy- und Polyhydroxy- di-, tri- und polycarbonsäuren gemeinsam mit dem Wirkstoff (A) einzusetzen. Hierbei hat sich gezeigt, daß neben den Hydroxycarbonsäuren auch die Hydroxycarbonsäureester sowie die Mischungen aus Hydroxycarbonsäuren und deren Estern als auch polymere Hydroxycarbonsäuren und deren Ester ganz besonders bevorzugt sein können. Bevorzugte Hydroxycarbonsäureester sind beispielsweise Vollester der Glycolsäure, Milchsäure, Äpfelsäure, Weinsäure oder Citronensäure. Weitere grundsätzlich geeigneten Hydroxycarbonsäureester sind Ester der β-Hydroxypropionsäure, der Tartronsäure, der D-Gluconsäure, der Zuckersäure, der Schleimsäure oder der Glucuronsäure. Als Alkoholkomponente dieser Ester eignen sich primäre, lineare oder verzweigte aliphatische Alkohole mit 8 - 22 C-Atomen, also z.B. Fettalkohole oder synthetische Fettalkohole. Dabei sind die Ester von C12-C15-Fettalkoholen besonders bevorzugt. Ester dieses Typs sind im Handel erhältlich, z.B. unter dem Warenzeichen Cosmacol® der EniChem, Augusta Industriale. Besonders bevorzugte Polyhydroxypolycarbonsäuren sind Polymilchsäure und Polyweinsäure sowie deren Ester.

Weitere Gegenstände der vorliegenden Erfindung sind die Verwendung von Mischungen aus
A) einer oder mehrerer als Konservierungsmittel wirksamer organischer Säure(n) und/oder physiologisch verträglicher wasserlöslicher Salze dieser Säuren,
B) Homo- oder Copolymeren aus Acrylsäure und/oder Methacrylsäure und/oder Estern dieser Säuren, welche mit Allylethern von Sucrose und/oder Allylethern von Pentaerythrit vernetzt sind
zur Viskositätsregulierung wäßriger vorzugsweise kosmetischer Zusammensetzungen, insbesondere von Haarreinigungsmitteln und die Verwendung von Mischungen aus
A) einer oder mehrerer als Konservierungsmittel wirksamer organischer Säure(n) und/oder physiologisch verträglicher wasserlöslicher Salze dieser Säuren,
B) Homo- oder Copolymeren aus Acrylsäure und/oder Methacrylsäure und/oder Estern dieser Säuren, welche mit Allylethern von Sucrose und/oder Allylethern von Pentaerythrit vernetzt sind
zur Temperaturstabilisierung der Viskosität wäßriger vorzugsweise kosmetischer Zusammensetzungen, insbesondere von Haarreinigungsmitteln.

Bezüglich bevorzugter erfindungsgemäßer Verwendungen gilt mutatis mutandis das zu bevorzugten erfindungsgemäßen Mitteln Gesagte.

## Patentansprüche

1. Haarreinigungsmittel, enthaltend bezogen auf sein Gewicht
a) 5 bis 50 Gew.-% Tensid(e);
b) 0,01 bis 5 Gew.-% einer oder mehrerer als Konservierungsmittel wirksamer organischer Säure(n) aus der Gruppe Salicylsäure, Benzoesäure, Propionsäure, Dehydracetsäure (3-Acetyl-6-methyl-2H-pyran-2,4(3H)-dion) und Sorbinsäure sowie den physiologisch verträglichen wasserlöslichen Salzen dieser Säuren;
c) 0,1 bis 5 Gew.-% mindestens eines Homo- oder Copolymers aus Acrylsäure und/oder Methacrylsäure und/oder Estern dieser Säuren, welches mit Allylethern von Sucrose und/oder Allylethern von Pentaerythrit vernetzt ist,
wobei das Mittel - abgesehen von der Komponente b) - frei von Konservierungsmitteln ist,
wobei die als Konservierungsmittel wirksame Säure ausgewählt ist aus Salicylsäure und ihren physiologisch verträglichen wasserlöslichen Salzen, und
wobei es einen pH-Wert von 4,5 bis 5,5 aufweist.

2. Haarreinigungsmittel nach Anspruch 1, **dadurch gekennzeichnet, daß** es einen pH-Wert von 4,8 bis 5,2, weiter bevorzugt von 4,9 bis 5,1 und insbesondere von 5,0 aufweist.

3. Haarreinigungsmittel nach einem der Ansprüche 1 oder 2, **dadurch gekennzeichnet, daß** es zusätzlich 0,05 bis 10 Gew.-%, vorzugsweise 0,1 bis 5 Gew.-%, besonders bevorzugt 0,2 bis 4 Gew.-% und insbesondere 0,3 bis 3 Gew.-% kationische(s) Polymer(e), vorzugsweise kationische(s) Cellulosederivat(e) und/oder kationische(s) Guar-Derivat(e), insbesondere Polyquaternium-10, enthält.

4. Haarreinigungsmittel nach einem der Ansprüche 1 bis 3, **dadurch gekennzeichnet, daß** es die Komponente(n) b) in Mengen von 0,05 bis 4 Gew.-%, vorzugsweise von 0,3 bis 3 Gew.-%, besonders bevorzugt von 0,4 bis 2 Gew.-% und insbesondere von 0,5 bis 1 Gew.-%, jeweils bezogen auf das gesamte Mittel, enthält.

5. Haarreinigungsmittel nach einem der Ansprüche 1 bis 4, **dadurch gekennzeichnet, daß** es als Komponete c) ein Homopolymer von Acrylsäure enthält, das mit Allylethern von Sucrose und/oder Allylethern von Pentaerythrit vernetzt ist (INCI-Bezeichnung: Carbomer).

6. Haarreinigungsmittel nach einem der Ansprüche 1 bis 5, **dadurch gekennzeichnet, daß** es als Komponente c) ein Copolymer aus
i. Acrylsäure und/oder Methacrylsäure und/oder Estern dieser Säuren und
ii. Alkylacrylat(en)
enthält, das mit Allylethern von Sucrose und/oder Allylethern von Pentaerythrit vernetzt ist (INCI-Bezeichnung: Acrylates/Alkyl Acrylate Crospolymer).

7. Haarreinigungsmittel nach Anspruch 6, **dadurch gekennzeichnet, daß** es als Komponente c) ein Copolymer aus
i. Acrylsäure und/oder Methacrylsäure und/oder Estern dieser Säuren und
ii. C₁₀₋₃₀-Alkylacrylat(en)
enthält, das mit Allylethern von Sucrose und/oder Allylethern von Pentaerythrit vernetzt ist (INCI-Bezeichnung: Acrylates/C10-30 Alkyl Acrylate Crospolymer).

8. Haarreiningungsmittel nach einem der Ansprüche 1 bis 7, **dadurch gekennzeichnet, daß** es die Komponente(n) c) in Mengen von 0,2 bis 4 Gew.-%, vorzugsweise von 0,3 bis 3 Gew.-%, besonders bevorzugt von 0,4 bis 2,5 Gew.-% und insbesondere von 0,5 bis 1,5 Gew.-%, jeweils bezogen auf das gesamte Mittel, enthält.

9. Haarreinigungsmittel nach einem der Ansprüche 1 bis 8, **dadurch gekennzeichnet, daß** es zusätzlich mindestens ein Silikon der Formel I
**(CH₃)₃Si-[O-Si(CH₃)₂]ₓ-O-Si(CH₃)₃** **(I),**
enthält, in der x für eine Zahl von 0 bis 100, vorzugsweise von 0 bis 50, weiter bevorzugt von 0 bis 20 und insbesondere 0 bis 10, steht.

10. Haarreinigungsmittel nach einem der Ansprüche 1 bis 9, **dadurch gekennzeichnet, daß** es ein aminofunktionelles Silikon der Formel II
**R'ₐG₃₋ₐ-Si(OSiG₂)ₙ-(OSiG_{b}R'_{2-b})ₘ-O-SiG₃₋ₐ-R'ₐ** **(II),**
enthält, worin bedeutet:
- G ist-H, eine Phenylgruppe, -OH, -O-CH₃, -CH₃, -O-CH₂CH₃, -CH₂CH₃, -O-CH₂CH₂CH₃,-CH₂CH₂CH₃, -O-CH(CH₃)₂, -CH(CH₃)₂, -O-CH₂CH₂CH₂CH₃, -CH₂CH₂CH₂CH₃, -O-CH₂CH(CH₃)₂, -CH₂CH(CH₃)₂, -O-CH(CH₃)CH₂CH₃, -CH(CH₃)CH₂CH₃, -O-C(CH₃)₃, -C(CH₃)₃ ;
- a steht für eine Zahl zwischen 0 und 3, insbesondere 0;
- b steht für eine Zahl zwischen 0 und 1, insbesondere 1,
- m und n sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R' ist ein monovalenter Rest ausgewählt aus
∘ -Q-N(R")-CH₂-CH₂-N(R")₂
∘ -Q-N(R")₂
∘ -Q-N⁺(R")₃A⁻
∘ -Q-N⁺H(R")₂A⁻
∘ -Q-N⁺H₂(R")A⁻
∘ -Q-N(R")-CH₂-CH₂-N⁺R"H₂A⁻,
wobei jedes Q für eine chemische Bindung, -CH₂-, -CH₂-CH₂-, -CH₂CH₂CH₂-, -C(CH₃)₂-, -CH₂CH₂CH₂CH₂-, -CH₂C(CH₃)₂-, -CH(CH₃)CH₂CH₂- steht,
R" für gleiche oder verschiedene Reste aus der Gruppe -H, -Phenyl, -Benzyl, -CH₂-CH(CH₃)Ph, der C₁₋₂₀-Alkylreste, vorzugsweise -CH₃, -CH₂CH₃, -CH₂CH₂CH₃, -CH(CH₃)₂, -CH₂CH₂CH₂H₃, -CH₂CH(CH₃)₂, -CH(CH₃)CH₂CH₃, -C(CH₃)₃, steht und A ein Anion repräsentiert, welches vorzugsweise ausgewählt ist aus Chlorid, Bromid, Iodid oder Methosulfat.

11. Haarreinigungsmittel nach einem der Ansprüche 1 bis 10, **dadurch gekennzeichnet, daß** es mindestens ein Silikon der Formel III 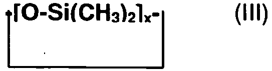 enthält, in der x für eine Zahl von 0 bis 200, vorzugsweise von 0 bis 10, weiter bevorzugt von 0 bis 7 und insbesondere 0, 1, 2, 3, 4, 5 oder 6, steht.

12. Haarreinigungsmittel nach einem der Ansprüche 1 bis 11, **dadurch gekennzeichnet, daß** es mindestens ein Silikon der Formel IV
**R₃Si-[O-SiR₂]ₓ-(CH₂)ₙ-[O-SiR₂]_{y}-O-SiR₃** **(IV),**
enthält, in der R für gleiche oder verschiedene Reste aus der Gruppe -H, - Phenyl, -Benzyl, -CH₂-CH(CH₃)Ph, der C₁₋₂₀-Alkylreste, vorzugsweise -CH₃, - CH₂CH₃, -CH₂CH₂CH₃, -CH(CH₃)₂, -CH₂CH₂CH₂H₃, -CH₂CH(CH₃)₂, -CH(CH₃)CH₂CH₃, -C(CH₃)₃, steht, x bzw. y für eine Zahl von 0 bis 200, vorzugsweise von 0 bis 10, weiter bevorzugt von 0 bis 7 und insbesondere 0, 1, 2, 3, 4, 5 oder 6, stehen, und n für eine Zahl von 0 bis 10, bevorzugt von 1 bis 8 und insbesondere für 2, 3, 4, 5, 6 steht.

13. Haarreinigungsmittel nach einem der Ansprüche 1 bis 12, **dadurch gekennzeichnet, daß** es mindestens ein wasserlösliches Silikon enthält.

14. Haarreinigungsmittel nach einem der Ansprüche 1 bis 13, **dadurch gekennzeichnet, daß** es - bezogen auf sein Gewicht - 5 bis 45 Gew.-%, vorzugsweise 6 bis 42 Gew.-% und insbesondere 7,5 bis 25 Gew.-% anionische(s) und/oder nichtionische(s) und/oder kationische(s) und/oder amphotere(s) Tensid(e), enthält.

15. Haarreinigungsmittel nach einem der Ansprüche 1 bis 14, **dadurch gekennzeichnet, daß** es Vitamine, Pro-Vitamine und Vitaminvorstufen enthält, die den Gruppen A, B, C, E, F und H zugeordnet werden, wobei bevorzugte Zusammensetzungen die genannten Verbindungen in Mengen von von 0,1 bis 5 Gew.-%, vorzugsweise von 0,25 bis 4 Gew.-% und insbesondere von 0,5 bis 2,5 Gew.-%, jeweils bezogen auf das gesamte Mittel, enthalten.

## Claims

1. A hair cleansing agent containing, relative to the weight thereof,
a) 5 to 50 wt.% of surfactant(s);
b) 0.01 to 5 wt.% of one or more organic acid(s) with a preservative action from the group salicylic acid, benzoic acid, propionic acid, dehydracetic acid (3-acetyl-6-methyl-2H-pyran-2,4(3H)-dione) and sorbic acid together with the physiologically acceptable water-soluble salts of these acids;
c) 0.1 to 5 wt.% of at least one homo- or copolymer of acrylic acid and/or methacrylic acid and/or esters of these acids which is crosslinked with allyl ethers of sucrose and/or allyl ethers of pentaerythritol,
wherein the agent, apart from component b), contains no preservatives and wherein
the acid with a preservative action is selected from salicylic acid and the physiologically acceptable water-soluble salts thereof,
and wherein the agent exhibits a pH value of 4.5 to 5.5

2. A hair cleansing agent according to claim 1, **characterised in that** it exhibits a pH value of 4.8 to 5.2, more preferably of 4.9 to 5.1 and in particular of 5.0.

3. A hair cleansing agent according to either of claim 1 or claim 2, **characterised in that** it additionally contains 0.05 to 10 wt.%, preferably 0.1 to 5 wt.%, particularly preferably 0.2 to 4 wt.% and in particular 0.3 to 3 wt.% of cationic polymer(s), preferably cationic cellulose derivative(s) and/or cationic guar derivative(s), in particular Polyquaternium 10.

4. A hair cleansing agent according to any one of claims 1 to 3, **characterised in that** it contains component(s) b) in quantities of 0.05 to 4 wt.%, preferably of 0.3 to 3 wt.%, particularly preferably of 0.4 to 2 wt.% and in particular of 0.5 to 1 wt.%, in each case relative to the entire agent.

5. A hair cleansing agent according to any one of claims 1 to 4, **characterised in that**, as component c), it contains a homopolymer of acrylic acid which is crosslinked with allyl ethers of sucrose and/or allyl ethers of pentaerythritol (INCI name: Carbomer).

6. A hair cleansing agent according to any one of claims 1 to 5, **characterised in that**, as component c), it contains a copolymer of
i. acrylic acid and/or methacrylic acid and/or esters of these acids and
ii. alkyl acrylate(s)
which is crosslinked with allyl ethers of sucrose and/or allyl ethers of pentaerythritol (INCI name: Acrylates/Alkyl Acrylate Crospolymer).

7. A hair cleansing agent according to claim 6, **characterised in that**, as component c), it contains a copolymer of
i. acrylic acid and/or methacrylic acid and/or esters of these acids and
ii. C₁₀₋₃₀ alkyl acrylate(s)
which is crosslinked with allyl ethers of sucrose and/or allyl ethers of pentaerythritol (INCI name: Acrylates/C10-30 Alkyl Acrylate Crospolymer).

8. A hair cleansing agent according to any one of claims 1 to 7, **characterised in that** it contains component(s) c) in quantities of 0.2 to 4 wt.%, preferably of 0.3 to 3 wt.%, particularly preferably of 0.4 to 2.5 wt.% and in particular of 0.5 to 1.5 wt.%, in each case relative to the entire agent.

9. A hair cleansing agent according to any one of claims 1 to 8, **characterised in that** it additionally contains at least one silicone of formula I
(CH₃)₃Si-[O-Si(CH₃)₂]ₓ-O-Si(CH₃)₃ (I),
in which x denotes a number from 0 to 100, preferably from 0 to 50, more preferably from 0 to 20 and in particular from 0 to 10.

10. A hair cleansing agent according to any one of claims 1 to 9, **characterised in that** it contains an amino-functional silicone of formula II
R'ₐG₃₋ₐ-Si(OSiG₂)ₙ-(OSiG_{b}R'_{2-b})ₘ-O-SiG₃₋ₐ-R'ₐ (II),
in which:
- G is -H, a phenyl group, -OH, -O-CH₃, -CH₃, -O-CH₂CH₃, -CH₂CH₃, -O-CH₂CH₂CH₃, -CH₂CH₂CH₃, -O-CH(CH₃)₂, -CH(CH₃)₂, -O-CH₂CH₂CH₂CH₃, -CH₂CH₂CH₂CH₃, -O-CH₂CH(CH₃)₂, -CH₂CH(CH₃)₂, -O-CH(CH₃)CH₂CH₃, -CH(CH₃)CH₂CH₃, -O-C(CH₃)₃, -C(CH₃)₃;
- a denotes a number between 0 and 3, in particular 0;
- b denotes a number between 0 and 1, in particular 1,
- m and n are numbers, the sum of which (m + n) amounts to between 1 and 2000, preferably between 50 and 150, with n preferably assuming values from 0 to 1999 and in particular from 49 to 149 and m preferably assuming values from 1 to 2000, in particular from 1 to 10,
- R' is a monovalent residue selected from
∘ -Q-N(R")-CH₂-CH₂-N(R")₂
∘ -Q-N(R")₂
∘ -Q-N⁺(R")₃A⁻
∘ -Q-N⁺H(R")₂A⁻
∘ -Q-N⁺H₂(R")A⁻
∘ -Q-N(R")-CH₂-CH₂-N⁺R"H₂A⁻,
wherein each Q denotes a chemical bond, -CH₂-, -CH₂CH₂-, -CH₂CH₂CH₂-, -C(CH₃)₂-, -CH₂CH₂CH₂CH₂-, -CH₂C(CH₃)₂-, -CH(CH₃)CH₂CH₂-,
R" denotes identical or different residues from the group -H, phenyl, benzyl, -CH₂-CH(CH₃)Ph, C₁₋₂₀ alkyl residues, preferably -CH₃, -CH₂CH₃, -CH₂CH₂CH₃, -CH(CH₃)₂, -CH₂CH₂CH₂CH₃, -CH₂CH(CH₃)₂, -CH(CH₃)CH₂CH₃, -C(CH₃)₃, and A represents an anion which is preferably selected from chloride, bromide, iodide or methosulfate.

11. A hair cleansing agent according to any one of claims 1 to 10, **characterised in that** it contains at least one silicone of formula III 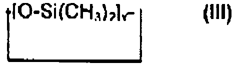 in which x denotes a number 3, 4, 5 or 6.

12. A hair cleansing agent according to any one of claims 1 to 11, **characterised in that** it contains at least one silicone of formula IV
R₃Si-[O-SiR₂]ₓ(CH₂)ₙ-[O-SiR₂]_{y}-O-SiR₃ (IV),
in which R denotes identical or different residues from the group -H, phenyl, benzyl, -CH₂-CH(CH₃)Ph, C₁₋₂₀ alkyl residues, preferably -CH₃, -CH₂CH₃, -CH₂CH₂CH₃, -CH(CH₃)₂, -CH₂CH₂CH₂CH₃, -CH₂CH(CH₃)₂, CH(CH₃)CH₂CH₃, -C(CH₃)₃, x and/or y denote a number from 0 to 200, preferably from 0 to 10, more preferably from 0 to 7 and in particular 0, 1, 2, 3, 4, 5 or 6, and n denotes a number from 0 to 10, more preferably from 1 to 8 and in particular 2, 3, 4, 5, 6.

13. A hair cleansing agent according to any one of claims 1 to 12, **characterised in that** it contains at least one water-soluble silicone.

14. A hair cleansing agent according to any one of claims 1 to 13, **characterised in that**, relative to the weight thereof, it contains 5 to 45 wt.%, preferably 6 to 42 wt.% and in particular 7.5 to 25 wt.% of anionic and/or nonionic and/or cationic and/or amphoteric surfactant(s).

15. A hair cleansing agent according to any one of claims 1 to 14, **characterised in that** it contains vitamins, pro-vitamins and vitamin precursors which are assigned to groups A, B, C, E, F and H, wherein preferred compositions contain the stated compounds in quantities of 0.1 to 5 wt.%, preferably of 0.25 to 4 wt.% and in particular of 0.5 to 2.5 wt.%, in each case relative to the entire agent.

## Revendications

1. Agent de nettoyage capillaire contenant, rapportés à son poids :
a) de 5 à 50 % en poids d'agent(s) tensioactif(s) ;
b) de 0,01 à 5 % en poids d'un ou de plusieurs acides organiques faisant office de conservateurs choisis parmi le groupe comprenant l'acide salicylique, l'acide benzoïque, l'acide propionique, l'acide déshydroacétique (3-acétyl-6-méthyl-2H-pyrane-2,4(3H)-dione) et l'acide sorbique, ainsi que les sels hydrosolubles de ces acides, physiologiquement acceptables ;
c) de 0,1 à 5 % en poids d'au moins un homopolymère ou un copolymère d'acide acrylique et/ou d'acide méthacrylique et/ou d'esters de ces acides, qui est réticulé avec des éthers alkyliques de saccharose et/ou des éthers allyliques du pentaérythritol ;
dans lequel l'agent - indépendamment du composant b) - est exempt de conservateurs, et
l'acide faisant office de conservateur est choisi parmi l'acide salicylique et ses sels hydrosolubles physiologiquement acceptables, et
dans lequel l'agent présente une valeur de pH de 4,5 à 5,5.

2. Agent de nettoyage capillaire selon la revendication 1, **caractérisé en ce qu'**il présente une valeur de pH de 4,8 à 5,2, de préférence de 4,9 à 5,1, et en particulier de 5,0.

3. Agent de nettoyage capillaire selon la revendication 1 ou 2, **caractérisé en ce qu'**il contient en outre de 0,05 à 10 % en poids, de préférence de 0,1 à 5 % en poids, de manière particulièrement préférée de 0,2 à 4 % en poids, et en particulier de 0,3 à 3 % en poids de polymère(s) cationique(s), de préférence de dérivé(s) cationique(s) de cellulose et/ou de dérivé(s) cationique(s) de la gomme de Guar, en particulier du Polyquaternium-10.

4. Agent de nettoyage capillaire selon l'une quelconque des revendications 1 à 3, **caractérisé en ce qu'**il contient le ou les composant(s) b) dans des quantités de 0,05 à 4 % en poids, de préférence de 0,3 à 3 % en poids, de manière particulièrement préférée de 0,4 à 2 % en poids et en particulier de 0,5 à 1 % en poids, chaque fois rapportés à l'agent dans sa totalité.

5. Agent de nettoyage capillaire selon l'une quelconque des revendications 1 à 4, **caractérisé en ce qu'**il contient, à titre de composant c), un homopolymère d'acide acrylique qui est réticulé avec des éthers allyliques du saccharose et/ou des éthers allyliques du pentaérythritol (appellation INCI : Carbomer).

6. Agent de nettoyage capillaire selon l'une quelconque des revendications 1 à 5, **caractérisé en ce qu'**il contient, à titre de composant c), un copolymère
i) d'acide acrylique et/ou d'acide méthacrylique et/ou d'esters de ces acides, et
ii) d'acrylate(s) d'alkyle,
qui est réticulé avec des éthers allyliques du saccharose et/ou des éthers allyliques du pentaérythritol (appellation INCI : Acrylates/Alkyl Acrylate Crospolymer).

7. Agent de nettoyage capillaire selon la revendication 6, **caractérisé en ce qu'**il contient, à titre de composant c), un copolymère
i) d'acide acrylique et/ou d'acide méthacrylique et/ou d'esters de ces acides, et
ii) d'acrylate(s) d'alkyle en C₁₀-C₃₀,
qui est réticulé avec des éthers allyliques du saccharose et/ou des éthers allyliques du pentaérythritol (appellation INCI : Acrylates/C10-30 Alkyl Acrylate Crospolymer).

8. Agent de nettoyage capillaire selon l'une quelconque des revendications 1 à 7, **caractérisé en ce qu'**il contient le ou les composant(s) c) dans des quantités de 0,2 à 4 % en poids, de préférence de 0,3 à 3 % en poids, de manière particulièrement préférée de 0,4 à 2,5 % en poids et en particulier de 0,5 à 1,5 % en poids, chaque fois rapportés à l'agent dans sa totalité.

9. Agent de nettoyage capillaire selon l'une quelconque des revendications 1 à 8, **caractérisé en ce qu'**il contient en outre au moins une silicone répondant à la formule (I)
(CH₃)₃Si-[O-Si(CH₃)₂]ₓ-O-Si(CH₃)₃ (I),
dans laquelle x représente un nombre de 0 à 100, de préférence de 0 à 50, de manière plus préférée de 0 à 20 et en particulier de 0 à 10.

10. Agent de nettoyage capillaire selon l'une quelconque des revendications 1 à 9, **caractérisé en ce qu'**il contient une silicone aminofonctionnelle répondant à la formule (II)
R'ₐG₃₋ₐ-Si(OSiG₂)ₙ-(OSiG_{b}R'_{2-b})ₘ-O-SiG₃₋ₐ-R'ₐ (II),
dans laquelle :
- G représente un atome d'hydrogène, un groupe phényle, un groupe -OH, un groupe -O-CH₃, un groupe -CH₃, un groupe -O-CH₂CH₃, un groupe -CH₂CH₃, un groupe -OCH₂CH₂CH₃, un groupe -CH₂CH₂CH₃, un groupe -O-CH(CH₃)₂, un groupe -CH(CH₃)₂, un groupe -O-CH₂CH₂CH₂CH₃, un groupe -CH₂CH₂CH₂CH₃, un groupe -O-CH₂CH(CH₃)₂, un groupe -CH₂CH(CH₃)₂, un groupe -O-CH(CH₃)CH₂CH₃, un groupe -CH(CH₃)CH₂CH₃, un groupe -O-C(CH₃)₃, un groupe -C(CH₃)₃;
- a représente un nombre entre 0 et 3, en particulier 0 ;
- b représente un nombre entre 0 et 1, en particulier 1 ;
- m et n représentent un nombre dont la somme (m + n) s'élève entre 1 et 2000, de préférence entre 50 et 150, n prenant de préférence des valeurs de 0 à 1999 et en particulier de 49 à 149, et m prenant de préférence des valeurs de 1 à 2000, en particulier de 1 à 10;
- R' représente un résidu monovalent choisi parmi :
-Q-N(R")-CH₂-CH₂-N(R")₂
-Q-N(R")₂
-Q-N⁺(R")₃A⁻
-Q-N⁺H(R")₂A⁻
-Q-N⁺H₂(R")A⁻
-Q-N(R")-CH₂-CH₂-N⁺R"H₂A⁻,
chaque résidu Q représentant une liaison chimique, un groupe -CH₂-, un groupe -CH₂-CH₂-, un groupe -CH₂CH₂CH₂, un groupe -C(CH₃)₂-, un groupe -CH₂CH₂CH₂CH₂-, un groupe -CH₂C(CH₃)₂-, un groupe -CH(CH₃)CH₂CH₂- ;
R" représentant des résidus identiques ou différents choisis parmi le groupe comprenant un atome d'hydrogène, un groupe phényle, un groupe benzyle, un groupe -CH₂-CH(CH₃)Ph, des résidus alkyle en C₁-C₂₀, de préférence un résidu -CH₃, un résidu -CH₂CH₃, un résidu -CH₂CH₂CH₃, un résidu -CH(CH₃)₂, un résidu -CH₂CH₂CH₂CH₃, un résidu -CH₂CH(CH₃)₂, un résidu -CH(CH₃)CH₂CH₃, un résidu -C(CH₃)₃, et A représente un anion qui est choisi de préférence parmi un chlorure, un bromure, un iodure ou un méthosulfate.

11. Agent de nettoyage capillaire selon l'une quelconque des revendications 1 à 10, **caractérisé en ce qu'**il contient au moins une silicone répondant à la formule (III) 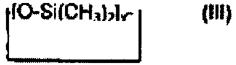 dans laquelle x représente un nombre égal à 3, 4, 5 ou 6.

12. Agent de nettoyage capillaire selon l'une quelconque des revendications 1 à 11, **caractérisé en ce qu'**il contient au moins une silicone répondant à la formule (IV)
R₃Si-[O-SiR₂]ₓ(CH₂)ₙ-[O-SiR₂]_{y}-O-SiR₃ (IV),
dans laquelle R représente des résidus identiques ou différents choisis parmi le groupe comprenant un atome d'hydrogène, un groupe phényle, un groupe benzyle, un groupe -CH₂-CH(CH₃)Ph, des résidus alkyle en C₁-C₂₀, de préférence un résidu -CH₃, un résidu -CH₂CH₃, un résidu -CH₂CH₂CH₃, un résidu -CH(CH₃)₂, un résidu -CH₂CH₂CH₂CH₃, un résidu -CH₂CH(CH₃)₂, un résidu -CH(CH₃)CH₂CH₃, un résidu -C(CH₃)₃, x respectivement y représente un nombre de 0 à 200, de préférence de 0 à 10, de manière plus préférée de 0 à 7, et en particulier s'élevant à 0, 1, 2, 3, 4, 5 ou 6, et n représente un nombre de 0 à 10, de préférence de 1 à 8, et en particulier s'élevant à 2, 3, 4, 5, 6.

13. Agent de nettoyage capillaire selon l'une quelconque des revendications 1 à 12, **caractérisé en ce qu'**il contient au moins une silicone hydrosoluble.

14. Agent de nettoyage capillaire selon l'une quelconque des revendications 1 à 13, **caractérisé en ce qu'**il contient - rapportés à son poids - de 5 à 45 % en poids, de préférence de 6 à 42 % en poids, et en particulier de 7,5 à 25 % en poids d'agent(s) tensioactif(s) anionique(s) et/ou non ionique(s) et/ou cationique(s) et/ou amphotère(s).

15. Agent de nettoyage capillaire selon l'une quelconque des revendications 1 à 14, **caractérisé en ce qu'**il contient des vitamines, des provitamines et des précurseurs de vitamines qui appartiennent aux groupes A, B, C, E, F et H, des compositions préférées contenant les composés mentionnés dans des quantités de 0,1 à 5 % en poids, de préférence de 0,25 à 4 % en poids et en particulier de 0,5 à 2,5 % en poids, chaque fois rapportés à l'agent dans sa totalité.
